(19) Europäisches Patentamt / European Patent Office / Office européen des brevets

(11) **EP 3 089 765 B1**

(12) **EUROPEAN PATENT SPECIFICATION**

(45) Date of publication and mention
of the grant of the patent:
**18.11.2020 Bulletin 2020/47**

(51) Int Cl.:
**A61L 15/28** *(2006.01)*    **A61L 15/42** *(2006.01)*

(21) Application number: **14828042.3**

(86) International application number:
**PCT/FI2014/051062**

(22) Date of filing: **30.12.2014**

(87) International publication number:
**WO 2015/101712 (09.07.2015 Gazette 2015/27)**

(54) **BIOMEDICAL DEVICE**

BIOMEDIZINISCHE VORRICHTUNG

DISPOSITIF BIOMÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2013 FI 20136336**

(43) Date of publication of application:
**09.11.2016 Bulletin 2016/45**

(73) Proprietor: **UPM-Kymmene Corporation**
**00100 Helsinki (FI)**

(72) Inventors:
• **LAUKKANEN, Antti**
**00250 Helsinki (FI)**
• **YLIPERTTULA, Marjo**
**02330 Espoo (FI)**
• **GANDIA-VENTURA, Carolina**
**46960 Aldaia, Valencia (ES)**
• **ESCOBEDO-LUCEA, Carmen**
**46024 Valencia (ES)**
• **PALTAKARI, Jouni**
**02250 Espoo (FI)**

• **BESSONOFF, Marko**
**02100 Espoo (FI)**

(74) Representative: **Boco IP Oy Ab**
**Itämerenkatu 5**
**00180 Helsinki (FI)**

(56) References cited:
**WO-A2-2012/056109    WO-A2-2013/171373**

• **YASUMITSU URAKI ET AL: "Fabrication of honeycomb-patterned cellulose material that mimics wood cell wall formation processes", MATERIALS SCIENCE AND ENGINEERING C, ELSEVIER SCIENCE S.A, CH, vol. 31, no. 6, 16 November 2010 (2010-11-16), pages 1201-1208, XP028223499, ISSN: 0928-4931, DOI: 10.1016/J.MSEC.2010.11.009 [retrieved on 2010-11-23]**
• **Molly M Stevens ET AL: "Exploring and Engineering the Cell Surface Interface", Science, vol. 310, no. 5751, 18 November 2005 (2005-11-18), pages 1135-1138, XP055376094, ISSN: 0036-8075, DOI: 10.1126/science.1106587**

**Description**

**FIELD OF THE INVENTION**

The present invention relates to the field of nanocellulose technology and biomedicine. In particular, the present invention relates to novel devices comprising cells and patterned membranes comprising nanofibrillar cellulose as defined in claim 1, and methods for manufacturing the devices, as defined in claim 18. Also disclosed are uses thereof in various applications, such as in wound treatment.

**BACKGROUND**

Treatment of skin wounds, particularly severe wounds and burns, is often difficult. Wound healing of the skin encompasses a series of cellular and molecular processes that act repairing the damaged tissue and re-establishing the barrier function of the skin. After injury, the initial response is the formation of a fibrin clot that prevents blood loss and infection. The released fibrin and chemokines attract neutrophils, macrophages, endothelial cells and fibroblast that act to prevent infection, form new blood vessels and synthesize extracellular matrix, respectively. Later, the clot barrier is replaced with migrating keratinocytes that repair wound surface and reconstitute new functional epithelium.

The healing process starts about 24 hours after injury and continues until the wound is recovered. Once new epithelium is established, the blood vessel density decreases in the wound area and the remodelling of the dermis continues for a period of several months. In cases of extreme injury as in severe burns or hypothermal injuries the skin may be so damaged that it is not able to repair the wound and sometimes the patient cannot survive. In some other cases, the normal wound healing process may fail and is trapped in a constant inflammatory state. Such problems often arise if the patient has already acquired a chronic disease which impairs normal healing process, such as diabetes.

An important component of the healing process in adult mammals is the stimulation of fibroblasts to generate extracellular matrix. Extracellular matrix constitutes a major component of the connective tissue which develops to repair a wound area. The repair process, however, is not perfect and the connective tissue is often fibrous and commonly forms connective tissue scars (fibrosis). Scars are composed of a connective tissue which is predominately a matrix of collagen types 1 and 3 and fibronectin. The scar may consist of collagen fibers in an abnormal organization (as seen in scars of the skin) or it may be an abnormal accumulation of connective tissue (as seen in scars of the central nervous system). Most scars consist of abnormally organized collagen and excess collagen.

Another difficulty related to wound healing is contraction, which is generally regarded as a natural and essential element of wound healing. However, in many cases excessive and uncontrolled wound contraction can be observed during the healing process leading into contraction induced fibrosis, which can lead e.g. to disfigurement and impaired mobility of joints or limbs.

Various agents, wound dressings and composites have been proposed in the art to improve skin wound healing and to prevent inflammation, fibrosis and scarring. Wound dressings and ointment gauzes are generally used as therapy for a skin defect reaching to an upper layer of dermis, such as a superficial dermal burn. When a skin defect reaches a lower layer of dermis, such as a deep dermal burn, a dermal burn or a decubitus in at least the second grade, self-reconstruction in a cutaneous tissue by proliferation of epidermal cells becomes problematic. These defects are typically treated by debriding a slough or an abnormal granulation tissue, reconstructing a normal granulation tissue by covering the defect with an allogeneic skin, xenogeneic skin, artificial silicon skin, skin replacement products, wound dressings or the like, and then reconstructing a skin by performing autologous split-thickness skin graft (STSG), or with whole skin grafts.

While the above considerations mainly apply to wound healing and inflammation in humans, it will be appreciated that the same problems can also occur in animals, particularly veterinary or domestic animals (e. g. horses, cattle, dogs, cats etc.). WO01/03750 discloses material comprising human extracellular matrix and the framework to which the stromal cells may attach.

WO 2013/171373 discloses a method for preparing a membrane from fibril cellulose.

WO2012/056109 discloses plant derived nanofibrilar cellulose membranes for use in wound treatment.

EP06742940 discloses treatment of wounds, such as fistulae, with adipose tissue derived stromal stem cells. The isolated cells are delivered with a syringe to the treatment site.

Methods for creating micro scale texture on nanofibrillar cellulose have used electrospun cellulose scaffolds and laser ablation to make a flat cellulose membrane which has regularly arranged pores in the otherwise smooth membrane (Rodriguez K. et al., Electrospun nanofibrous cellulose scaffolds with controlled microarchitecture, Carbohydrate Polymers, 2013).

Jones, Currie and Martin cover several systems for wound healing in their review (A guide to biological skin substitutes, British Journal of Plastic Surgery (2002), 55, 185-193) one of them being Laserskin, an upside-down membrane delivery system created from laser-perforated derivative of esterified hyaluronic acid onto which keratinocytes are seeded *in vitro* to populate the laser-drilled pores. The cell colonies then grow above and below the membrane. This system has been used for the treatment of vitiligo, as well as to resurface Integra.

## BRIEF DESCRIPTION OF THE INVENTION

Despite some progress in the field, there is still a need to provide controlled and easy delivery of therapeutic cells on the wound site to achieve enhanced wound healing and prevention of inflammation during wound healing and tissue repair.

An object is to provide a novel patterned membrane comprising nanofibrillar polysaccharides for medical applications.

Another object is to provide novel compositions, devices and methods for preventing or at least partially ameliorating inflammation during skin wound healing.

Another object is to develop a device for treating skin wounds. Preferably the device is a biomedical device.

The inventors have developed a method to manufacture a patterned membrane comprising nanofibrillar polysaccharide arranged in a continuous arrangement having micro-scale topography and comprising recesses and/or protrusions on at least one side of the membrane. The membrane was surprisingly useful as a component in a device comprising the membrane and stem cells. Said device was shown to significantly enhance wound healing and prevent inflammation during wound healing and tissue repair.

Micro patterns were found to be particular useful for manufacturing a device for wound treatment. By using the inventive patterned membrane the therapeutic cells could be spread evenly on the patterned structure of the membrane and transported to the wound site where the cells detached from the membrane in a viable state. The device enhanced wound healing significantly when applied on the wound treatment site.

The patterned membrane comprising nanofibrillar polysaccharide arranged in a continuous arrangement can be manufactured by a method comprising the steps of:

    a. providing nanofibrillar polysaccharide dispersion on a patterned filter with micro-scale topography comprising recesses and/or protrusions;

    b. raising the dry matter content of the polysaccharide dispersion by draining liquid from the nanofibrillar polysaccharide dispersion by the effect of alteredpressure through the patterned filter which is essentially impermeable to the fibrils of the nanofibrillar polysaccharide but permeable to the liquid to form a membrane sheet on the patterned filter,

    c. optionally drying the membrane while continuing removing the liquid from the nanofibrillar polysaccharide dispersion, and

    d. optionally removing the membrane from the patterned filter,

whereby a patterned membrane comprising nanofibrillar polysaccharide is obtained which has micro-scale recesses and/or protrusions in an inverse arrangement compared to the patterned filter with micro-scale recesses and/or protrusions.

Furthermore, the membrane comprising nanofibrillar polysaccharide arranged in a continuous arrangement and having at least on one side of the membrane at least one patterned area comprising micro-scale recesses and/or protrusions can be manufactured by a method comprising the step of providing nanofibrillar polysaccharide dispersion; and a step selected from the group consisting of

    a. casting the nanofibrillar polysaccharide dispersion on a casting support comprising at least one patterned area comprising micro-scale recesses and/or protrusions, drying, and removing the formed membrane comprising at least one patterned area comprising micro-scale recesses and/or protrusions in an inverse arrangement compared to the casting support; and

    b. forming the nanofibrillar polysaccharide dispersion into a membrane, etching at least one area of the membrane to provide at least one patterned area comprising micro-scale recesses and/or protrusions.

The patterned membrane comprising nanofibrillar polysaccharide is used as a component in a device, such as in a biomedical device. The device comprises cells and a membrane comprising nanofibrillar polysaccharide arranged in a continuous arrangement, at least one side of the membrane comprising at least one patterned area comprising micro-scale recesses and/or protrusions. Specifically, the device comprises therapeutically useful cells, the patterned membrane comprising plant-derived nanofibrillar polysaccharides, and the patterned area comprising a repeating pattern of units with at least one dimension from 1 to 500 micrometers along the plane of the membrane, wherein the therapeutically useful cells are on the patterned area of the membrane.

The membrane is useful for use in therapy, for use in the treatment of wounds, preferably skin wounds or skin burns, for use in preventing inflammation, immune rejection, or scar formation during recovery from dermal tissue damage.

The device is manufactured by providing therapeutically useful cells; absorbing a patterned membrane comprising nanofibrillar polysaccharides and having a micro-scale topography comprising recesses and/or protrusions with an aqueous medium; transferring the cells on the membrane; and culturing the cells in conditions allowing attachment of the cells on the membrane and maintenance or undifferentiated or differentiated growth. When the biomedical device is manufactured the therapeutically useful cells are spread on the patterned membrane such that the cells may settle on the patterned surface comprising recesses and/or protrusions, or even inside the recess on the membrane.

Said device is manufactured by providing therapeutically useful cells; absorbing a membrane comprising nanofibrillar polysaccharide arranged in a continuous arrangement, at least one side of the membrane comprising at least one patterned area comprising micro-scale recesses and/or protrusions with an aqueous medium; transferring the cells on the membrane; and incubating the cells in conditions allowing attachment of the cells on the membrane, and allowing maintenance or undifferentiated or differentiated growth of the cells.

Typical sizes of prokaryotic cells are ca. 1-5 $\mu$m and of eukaryotic cells ca. 10-100 $\mu$m. The membrane comprising nanofibrillar polysaccharide arranged in a continuous structure and a patterned area comprising micro-scale recesses and/or protrusions is particularly suitable for accommodating cells and facilitating adhesion, proliferation and alignment of the cells. The membrane comprising nanofibrillar polysaccharide and micro-scale recesses and/or protrusions enable optimal attachment of e.g. therapeutically useful cells to the membrane facilitating their practical delivery to a treatment site, without binding the cells too tightly thereby facilitating full contact between the cells and the site, and even detachment of the cells to the site when necessary. Nano- or macro-scale patterns, if used alone, i.e. in the absence of micro-scale recesses and/or protrusions, would not provide the necessary microenvironment. The nanofibrillar polysaccharides may also provide nano-scale topography of the same size scale as the cell receptors rather than the whole cells. The nanofibrillar polysaccharides provide also excellent absorbency for the membrane thereby facilitating incorporation of aqueous culture media and any additional agents for the benefit of the cells being delivered or the site being treated. The micro-scale topography facilitates maintaining membrane's surface moist. A membrane where the recesses do not extend through the entire thickness of the membrane have the additional effect that, when used in the device, cells stay substantially on the surface and thereby more cells can be contacted with and transferred to the site being treated. Through-holes may also decrease the mechanical properties such as tear strength of the membrane.

The device according to an embodiment is useful for use in therapy, for use in treatment of wound, for use in treatment of skin wound or skin burns, or for use in preventing inflammation, immune rejection and/or scar formation during recovery from dermal tissue damage.

Other aspects further relate to use of the inventive patterned membrane and/or the inventive device in tissue engineering, microfluidics, and microelectronics.

Other aspects further relate to a kit comprising the inventive patterned membrane, optionally an aqueous medium, and an instruction for use in tissue engineering, microfluidics, microelectronics.

Also disclosed is a method of treating wounds by applying the inventive patterned membrane with therapeutic cells on the wound treatment site, such as wound site of a patient having a skin wound.

## BRIEF DESCRIPTION OF DRAWINGS

**Figure 1** shows the method of manufacturing the patterned membranes according to one embodiment.

**Figure 2** shows the method of manufacturing the patterned membranes according to another embodiment.

**Figure 3** shows a pressing step according to a second embodiment of the method of manufacturing the patterned membranes.

**Figure 4** shows a drying step according to a third embodiment of the method of manufacturing the patterned membranes.

**Figure 5** shows SEM images of the micropatterned NFC membranes made with 1 micrometer filter cloth. Magnification: x100, **A**; x400 **B,** and x1200 **C.** The filter cloth used in the production (x400, **D**)

**Figure 6** shows SEM images of the micropatterned NFC membranes made with 10 micrometer filter cloth. Magnification: x100, **A;** x400 **B,** and x1200 **C.** The filter cloth used in the production (x400, **D**).

**Figure 7** shows SEM image of the 10 $\mu$m filter cloth **(A)** and partially overlapping inversed SEM image of the corresponding NFC membrane **(B).**

**Figure 8** shows SEM image of the NFC membrane made with 1 $\mu$m filter cloth with different tilt angles. tilt angle 0 °, x100 **A;** tilt angle 0 °, x300 **B;** tilt angle 45°, x100 **C;** tilt angle 45 °, x200 **D.**

**Figure 9** shows general scheme for the isolation and preparation of the cells before their delivery to the wound for the treatment.

**Figure 10** shows Scanning Electron micrography showing the differences on surface patterning and hASC cells. 700X Magnification.

**(A)** Smooth side of the membrane showing hASC trying to attach on the surface. See the morphology of the cells forming spheroids.

**(B)** Rough patterned membrane side. Detailed view of monolayer of hASC growing over the nanocellulose membrane.

**Figure 11** shows transmission electron microscopy of hASC cells cultured on different coating conditions on plastic **(A-C)** and nanocellulose membrane **(D-F).**
A-C. The cells cultured on plastic show the typical fibroblast-like morphology of hASC. Nucleus is compacted and we can appreciate lipid droplets eccentrical in the cytoplasm. These indicate that after one week in culture some of the cells have started to differentiate. Cells directly seeded over NFC membrane, show similar characteristics that the ones grown over plastic. Nucleus are more oval and mitochondria show normal cresta. **E.** The cells show elongated nuclei and well defined mitochondria. **F.** In the case of coating with cell start, the cells do not present any nuclear variation. At cytoplasm level increasing amount of lipid droplets can be observed in the cultures.

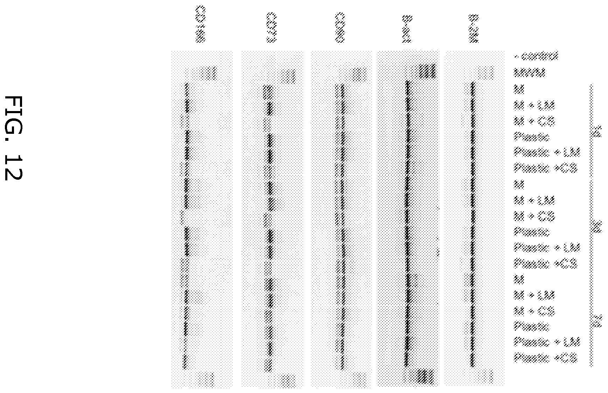

**Figure 12** presents agarose gel electrophoresis, showing the results from QRT_PCR for undifferentiation mesenchymal markers. After 1.3 and 7 days cultured over NFC membrane in the different conditions, hASC cells continue expressing mesenchymal undifferentiation markers in the same level that their counterparts cultured over plastic.

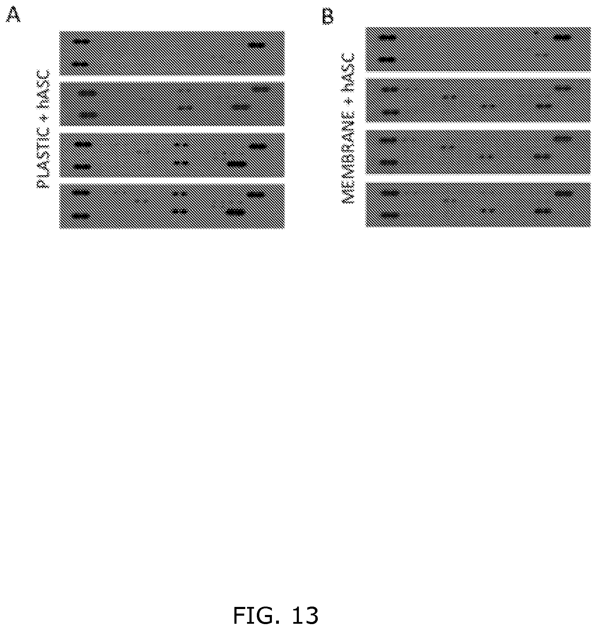

**Figure 13** presents Xray exposed film-showing differences in cytokine expression between hASC cells cultured with the different coatings over nanocellulose membrane versus plastic.

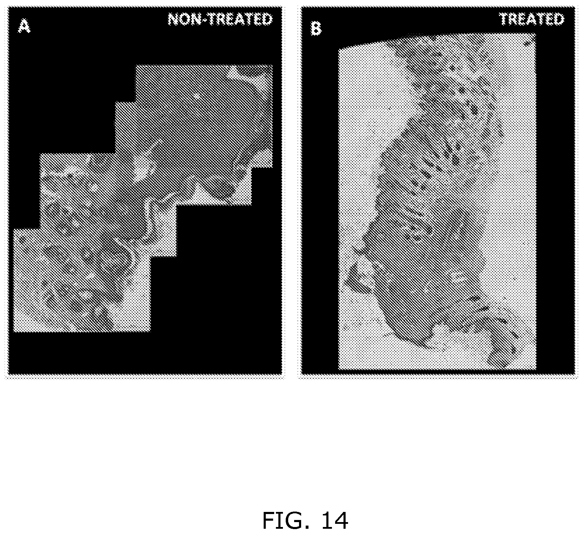

**Figure 14** shows pathology studies of control and treated animal, 5 days after nanocellulose membrane and cells treatment.

**(A).** Injury non-treated. This injury presents a traumatic area. The pink line in the border is a line of fibrin. This fibrin synthesis is tone of the first signals of wound healing response after injury. A lot of inflammatory cells are detected in Epidermis. This indicates that the injury is in the initial steps of wound healing recover. In deeper parts (down epidermis and initial dermis) extracellular matrix with a lot of fibroblast embedded is detected, which means that the wound is in an immature phase of recovering (at initial stages).

**(B).** Epidermis quite recovered and mature. Dermis is more immature and with traces of edema and inflammatory cells. Wound healing is faster in treated cells, especially in the layer that are in the surface and in contact with the apposite with cells The evolution of the dermis during the process needs to be followed. Hair follicles are well developed and organized. Dermis is in reconstruction and muscle not well organized yet.

## DETAILED DESCRIPTION OF THE INVENTION

Unless otherwise specified, the terms, which are used in the specification and claims, have the meanings commonly used in the field of cell culture or nanocellulose technology. Specifically, the following terms have the meanings indicated below.

As used herein, the term "polysaccharide" is understood to encompass long linear or branched carbohydrate molecules of repeated monomer units joined together by glycosidic bonds, and complex carbohydrates composed of a chain of monosaccharides joined together by glycosidic bonds. Non-limiting examples of polysaccharides according to the embodiments of the invention are cellulose, hemicellulose, chitin, chitosan, alginate, pectin, arabinoxylan, nanofibrillar cellulose, or derivatives thereof.

The term "nanofibril" refers to existing substructures isolated from the polysaccharide raw material. Here, the nanofibril does not refer to structures obtained by destroying the substructures of the polysaccharide raw material e.g. by dissolving and then creating a new structure, such as electrospun polysaccharides. The term "nanofibrillar polysaccharide" thus refers to a collection of polysaccharide nanofibrils or nanofibril bundles. As a non-limiting example the term "nanofibrillar polysaccharide" comprises "nanofibrillar cellulose", or "NFC", referring to all microfibrillated celluloses (MFC) and nanocelluloses. Further, there are several other widely used synonyms for NFC, for example fibril cellulose, cellulose nanofiber, nanofibrillated cellulose (CNF), nano-scale fibrillated cellulose, microfibrillar cellulose, or cellulose microfibrils.

Nanofibrillar cellulose comprises isolated cellulose microfibrils or microfibril bundles derived from cellulose raw material. Nanofibrillar cellulose is based on a natural polysaccharide polymer that is abundant in nature, especially in plants and in certain bacteria.

Production techniques of nanofibrillar cellulose are based on mechanical treatment by grinding or homogenization of aqueous dispersion of pulp fibers. The concentration of nanofibrillar cellulose in dispersions is typically very low, usually around 1-5 w%. After the grinding or homogenization process, the obtained nanofibrillar cellulose material is a dilute viscoelastic hydrogel.

Strong water retention is typical for nanofibrillar cellulose since water is bound to the fibrils through numerous hydrogen bonds. Consequently, reaching a dry matter content typical for membranes requires a long drying time and efficient water removal. Conventional methods such as vacuum filtration can take several hours to obtain a dry product. Low consistency of the fibrous polysaccharide dispersion favours formation of thin membranes with small variations in grammage over the surface of the membrane. On the other hand, this will increase the amount of water that has to be removed during drying.

With some nanofibrillar cellulose grades, such as nanofibrillar cellulose containing anionic groups (anionically charged nanofibrillar cellulose) the higher viscosity is an additional problem that causes longer dewatering times. Such anionically charged nanofibrillar cellulose can be for example chemically modified cellulose that contains carboxyl groups as a result of the modification. Cellulose obtained through N-oxyl mediated catalytic oxidation (e.g. through 2,2,6,6-tetramethyl-1-piperidine N-oxide) or carboxymethylated cellulose are examples of anionically charged nanofibrillar cellulose where the anionic charge is caused by the dissociated carboxylic acid moiety.

The term "continuous arrangement" refers to a structure or an arrangement, wherein nanofibrillar polysaccharides are present in a membrane as a continuous structure. In other words nanofibrillar polysaccharides are arranged along the whole membrane.

The term "aqueous medium" refers to any aqueous medium selected from the group consisting of water, sterile water, purified water, physiological saline, a physiological buffer, a culture medium, nutritional agents, and/or a bioactive agent, and combinations thereof. Aqueous medium may be any aqueous medium such as water, deionized water, buffer solution, or nutritional medium suitable for maintaining, transporting, isolating, culturing, propagating, passaging or differentiating of cells or tissues.

The term "interconnected" or "interconnection" refers to an arrangement wherein the membrane comprising nanofibrillar polysaccharide arranged in a continuous arrangement may comprise a patterned area having micro-scale recesses and/or protrusions as continuous interconnected units. In other words micro-scale recesses and/or protrusions are in contact with each other. The contact may be a direct connection between the recesses and/or protrusions or they may be loosely connected. In one aspect of the invention the patterned area may comprise a repeating pattern of units, which are interconnected with a common wall.

The term "micro-scale recesses and/or protrusions" refers to a topography with recesses and/or protrusions, where the recesses do not extend through the entire thickness of the membrane. The micro-scale recesses and/or protrusions provide a topography in the scale of typical cell sizes thereby facilitating adhesion, proliferation and alignment of the cells. The micro-scale topography facilitates maintaining membrane's surface moist. The micro-scale recesses and/or protrusions, provides the necessary microenvironment, which is not provided by nano- or macro-scale patterns, if used alone, i.e. in the absence of micro-scale recesses and/or protrusions.To obtain nanofibrillar cellulose, mechanical disintegration of cellulose pulp or oxidized cellulose raw material is carried out with suitable equipment such as a refiner, grinder, homogenizer, colloider, friction grinder, ultrasound-sonicator, fluidizer such as microfluidizer, macrofluidizer or fluidizer-type homogenizer. Preferably nanofibrillar cellulose is obtained using mechanical disintegration.

Several different grades of nanofibrillar cellulose have been developed using various production techniques. The grades have different properties depending on the manufacturing method, degree of fibrillation and chemical composition. The chemical compositions of the grades also vary. Depending on the raw material source, e.g. HW vs. SW pulp, different polysaccharide composition exists in the final cellulose nanofibril product. Typically, non-ionic or native grades have wider fibril diameter while the chemically modified grades are much thinner and have a continuous network.

The number average fibril diameter of the cellulose nanofibril is suitably from 1 to 200nm, preferably the number average fibril diameter of native grades is from 1 to 100nm, and in chemically modified grades from 1 to 20nm. Size distribution is also narrower for the modified grades. Native ion-exchanged cellulose nanofibrils exhibit discontinuous structure which is partially non-homogenous. In embodiments of the invention nanofibrillar cellulose is preferably non-toxic and sterile.

Derivatives of nanofibrillar cellulose can be any chemically or physically modified derivatives of cellulose that are suitable for the use in the invention, e.g. in cell culturing and in wound treatment. The chemical modification can be based for example on carboxymethylation, oxidation, esterification, or etherification reaction of cellulose molecules. Modification could also be realized by physical adsorption of anionic, cationic, or non-ionic substances or any combination of these on cellulose surface. The described modification can be carried out before, after, or during the production of nanofibrillar cellulose. Certain modifications may lead to materials that are degradable in human body.

Nanofibrillar cellulose and cellulose membranes according to the embodiments of the present invention can be synthetized or supplemented with agents that enhance wound healing, prevent scarring, or improve vascularization of the injured area.

The degree of substitution in the chemical derivatization process can vary broadly. For example, TEMPO or N-oxyl mediated oxidation is typically conducted to charge values from 300 to 1500 micromol/g, preferably 600 to 1200 micromol/g, most preferably 700 to 1100 micromol/g. The oxidized NFC may contain also aldehyde functional groups, typically between 0 to 250 micromol/g. Derivatization via carboxymethylation is typically conducted for cellulose pulp to ds levels between 0.05 to 0.3, preferably between 0.08-0.25, most preferably 0.10-0.2 prior to fibrillation. If the derivatization is conducted by cationization, the ds levels are typically between 0.05 and 0.4, preferably 0.15-0.3.

**Starting material of the membrane**

The nanofibrillar polysaccharide used as the starting material from which the patterned membrane is manufactured comprises plant-derived nanofibrillar cellulose. Preferably the nanofibrillar cellulose is at least partially composed of nanofibrillar cellulose, hemicellulose, chitin, chitosan, alginate, pectin, arabinoxylan, nanofibrillar cellulose, or derivatives thereof, most preferably the nanofibrillar cellulose is plant-derived nanofibrillar cellulose.

In one embodiment the nanofibrillar polysaccharide comprises nanofibrillar cellulose having the fibril diameter in the sub $\mu$m range. Nanofibrillar cellulose having this fibril diameter forms a self-assembled hydrogel network even at low concentrations. These gels of are highly shear thinning and thixotrophic in nature.

In one embodiment the nanofibrillar polysaccharide is native or unoxidised cellulose having the type 1 crystal structure or carboxymethylated cellulose at least partly having type 1 crystal structure.

The nanofibrillar polysaccharide is prepared from cellulose raw material of plant origin. The raw material can be based on any plant material that contains cellulose. Plant material may be wood. Wood can be from softwood tree such as spruce, pine, fir, larch, douglas-fir or hemlock, or from hardwood tree such as birch, aspen, poplar, alder, eucalyptus or acacia, or from a mixture of softwoods and hardwoods. Non-wood material can be from agricultural residues, grasses or other plant substances such as straw, leaves, bark, seeds, hulls, flowers, vegetables or fruits from cotton, corn, wheat, oat, rye, barley, rice, flax, hemp, manila hemp, sisal hemp, jute, ramie, kenaf, bagasse, bamboo or reed. The cellulose raw material could be also derived from cellulose-producing micro-organisms.

The term "nanofibrillar polysaccharide" and "fibril cellulose" refers to a collection of isolated microfibrils or microfibril bundles derived e.g. from cellulose raw material. Microfibrils have typically high aspect ratio: the length might exceed one micrometre while the number-average diameter is typically below 200 nm. The diameter of microfibril bundles can also be larger but generally less than 1 $\mu$m. The smallest microfibrils are similar to so called elementary fibrils, which are typically 2-12 nm in diameter. The dimensions of the fibrils or fibril bundles are dependent on raw material and disintegration method. The nanofibrillar cellulose may also contain some hemicelluloses; the amount is dependent on the plant source. Mechanical disintegration of nanofibrillar cellulose from cellulose raw material, cellulose pulp, or refined pulp is carried out with suitable equipment such as a refiner, grinder, homogenizer, colloider, friction grinder, ultrasound sonicator, fluidizer such as microfluidizer, macrofluidizer or fluidizer-type homogenizer.

One alternative is to obtain the fibrils from non-parenchymal plant material where the fibrils are obtained from secondary cell walls. One abundant source of cellulose fibrils is wood fibres. The nanofibrillated cellulose is manufactured by homogenizing wood-derived fibrous raw material, which may be chemical pulp. The disintegration in some of the above-mentioned equipment produces fibrils which have the diameter of only some nanometers, which is 50 nm at the most and gives a dispersion of fibrils in water. The fibrils can be reduced to size where the diameter of most of the fibrils is in the range of only 2-20 nm only. The fibrils originating in secondary cell walls are essentially crystalline with degree of crystallinity of at least 55 %.

The starting material for the patterned membrane preparation in embodiments of the invention is usually nanofibrillar cellulose obtained directly from the disintegration of some of the above mentioned fibrous raw material and existing at a relatively low concentration homogeneously distributed in water due to the disintegration conditions. The starting material can be an aqueous gel at a concentration of 0.05-5 w%. The gel of this type contains thus a great amount of water which is to be removed so that a network of cellulose fibrils forming the body of the membrane and causing the structural integrity and strength properties of the membrane is left. This network may contain other solids as well that were originally dispersed in the aqueous gel, but the cellulose fibrils are the main constituent of the membrane.

Nanofibrillar polysaccharide may comprise isolated nanofibrils and/or bundles formed of said nanofibrils. The smallest nanofibrils are similar to so called elementary fibrils, which are typically 2-12 nm in diameter. The dimensions of the nanofibrils or nanofibril bundles are dependent on raw material and disintegration method.

The number average diameter of nanofibrillar polysaccharide or nanofibrillar polysaccharide bundles may range between 1 and 500 nm, according to one suitable embodiment between 2 and 200 nm, according to another suitable embodiment between 2 and 100 nm, and according to a further suitable embodiment between 2 and 20 nm.

The number average diameter of native or non-derivatized nanofibrillar cellulose varies between 2-500 nm, preferably between 7 to 100 nm, and most preferably 7 to 50 nm. From Cryo-TEM images, also the bundled structure can be seen: the native grades are often mixtures of 7 nm elementary fibrils and 20-50 nm fibrillar bundles. The derivatized NFCs are typically thinner, the number average diameter varying between 2 to 200 nm, preferably 2 - 20 nm, most preferably 2-6 nm.

The length of nanofibrillar cellulose is somewhat challenging to measure accurately, but rough estimates for length of native grade is between 1 to 100 micrometer, preferably 1-50 micrometers, and most preferably 5-20 micrometers. The derivatized NFC are somewhat shorter; length varying between 0.3-50 micrometers, preferably 0.3-20 micrometers, and most preferably 0.5-10 micrometers. These values are estimated from CRYO-TEM, SEM or AFM images. The most accurate estimates are based on Cryo-TEM images.

Degree of fibrillation can be evaluated from fiber analysis where number of larger, only partially fibrillated, entities are evaluated. For example, in the case of derivatized nanofibrillar cellulose the number of those particles per mg of dry sample varies from 0 to 10000, preferably between 0 and 5000, most preferably between 0 and 1000. However, in non-derivatized NFC the number of non-fibrillated particles / mg is typically somewhat higher varying between 0 and 20000, preferably between 0 and 10000, and most preferably between 0 and 5000. The fiber analysis may suitably be carried out using FiberLab method as described below.

Fiber analysis - FiberLab method description

Commercial fiber analyzers may be used, and suitable devices are for example fiber analyzers Kajaani FiberLab or FS-300. The sample preparation and measurement is carried out as instructed for typical fiber coarseness -measurement, with the following exceptions: Dry matter content (DMC) is determined by weighing a sample mass of minimum 8 g for dry matter content determination, heating until constant weight.

Sample dilution is carried out as follows: Amount of sample to be diluted into 5 litre water vessel:

8 grams, if the DMC is around 2%.

16 grams, if the DMC is around 1%.

Pulp mixer is applied until all visible fibril bundles have disappeared.

Block removal -function is disabled.

A 50 ml sample is taken from the 5 litre vessel for the measurement. "Fibers per milligram" is calculated on the basis of the measurements:

$$FPM = ADF / (Mw * DMC/100 * Vp/Vv),$$

where

FPM = fiber per milligram [pcs/mg]

ADF = amount of fibers detected [pcs]

* This is the number of detected particles

Mw = amount of sample to be diluted into 5 litre water vessel [mg]

DMC = dry matter content of undiluted sample [%]

Vp = pipeted volume taken for the analyzer [ml]

Vv = volume of dilution vessel [ml].

The stiffness of the nanofibrillar polysaccharide hydrogels can be evaluated from viscoelastic measurements of the gels. Typically the storage modulus for 0.5% (by weight) nanofibrillar cellulose hydrogel in pure water at pH 7 at 25 °C is between 1 to 50 Pa, preferably 3 to 20 Pa. Often the derivatized NFC builds up stiffer hydrogels, but extensive fibrillation of these grades may lead also to lower storage modulus.

Rheological properties of nanofibrillar polysaccharide hydrogels can be also evaluated by monitoring viscosity as a function of shear stress or shear rate. The nanofibrillar polysaccharide hydrogels show plastic behaviour, which means that a certain shear stress (force) is required before the material starts to flow readily. This critical shear stress is often called the yield stress. The yield stress can be determined from a steady state flow curve measured with a stress controlled rheometer. When the viscosity is plotted as function of applied shear stress, a dramatic decrease in viscosity is seen after exceeding the critical shear stress. Zero-shear viscosity values varies typically between 1000 and 100 000 Pa s, preferably 5000 and 50 000 Pa s, in water at 0.5wt% concentration. For non-derivatized NFC the preferable range is between 1000 and 10 000 Pa s. The yield stress varies typically between 1 and 50 Pa s, preferably between 2 and 15 Pa s, in water at 0.5wt% concentration. Viscoelastic properties of nanofibrillar chitin and chitosan hydrogels resemble the situation with cellulose nanofiber hydrogels.

Rheological measurements of the NFC hydrogel are suitably carried out at room temperature at pH 7 with a stress controlled rotational rheometer (AR-G2, TA instruments, UK) equipped with four-bladed vane geometry. The diameters of the cylindrical sample cup and the vane are 30 mm and 28 mm, respectively. The length of the vane is 42 mm. The viscoelastic properties of the hydrogel are determined with a frequency sweep and a time sweep in dynamic oscillatory mode of the rheometer at a strain of 0.1 wt%. All samples are mixed, suitably with Waring blender prior to measurements (3 times 10 s).

Microbial purity of the nanofibrillar polysaccharide membranes according to an embodiment of the invention is essential for cell culture and medical applications. Therefore, the patterned membranes may be sterilized prior to cell culture or medical use. In addition to that it is important to minimize the microbial contamination of the product before and during the fibrillation. Prior to fibrillation, it is advantageous to aseptically collect the cellulose pulp from the pulp mill immediately after bleaching stage when the pulp is still sterile. Antimicrobial agents can be provided with the nanofibrillar polysaccharide according to the invention to prevent microbial growth.

**Liquid removal and pattern formation**

The patterned membrane comprising nanofibrillar polysaccharide can be manufactured by a method which simultaneously removes liquid from the dispersion and forms the patterned surface and which comprises the steps of

a. providing nanofibrillar polysaccharide dispersion on a patterned filter with micro-scale topography comprising recesses and/or protrusion;

b. draining liquid from the nanofibrillar polysaccharide dispersion by the effect of reduced pressure through the patterned filter which is impermeable to the fibrils of the nanofibrillar polysaccharide but permeable to the liquid to form a membrane web on the patterned filter,

c. optionally applying heat on the opposite side of the membrane web while continuing draining of the liquid through the patterned filter by pressure difference over the patterned filter, and

d. optionally removing the membrane web from the patterned filter as a freestanding nanofibrillar polysaccharide membrane, or, alternatively keeping the filter layer in the membrane as constituent layer of a membrane product comprising the filter layer and a nanofibrillar polysaccharide membrane;

whereby a membrane comprising nanofibrillar polysaccharide is obtained which has micro-scale topography comprising recesses and/or protrusions in an inverse arrangement of the patterned filter with micro-scale topography. Pattern formation is accomplished in the above method by removing water from the dispersion until the membrane is almost dry, whereby water-fibril bonds are replaced by fibril-fibril bonds that create an aggregated structure which is strong enough to remain essentially unchanged even when moistening the dried patterned membrane. The aggregation effect is especially significant for native nanofibrillar cellulose. Irreversible agglomeration of fibrils to large aggregates i.e. hornification is preferred. Hornification can occur during drying of aqueous suspensions of microfibrillar polysaccharides. It can be explained with the formation of a large number of hydrogen bonds between the hydroxyl groups of adjacent nanofibrils.

The optional heating step c. may be used to enhance water removal from the dispersion, but it is not required for patterning. Heat can be applied in step c. on the opposite side of the membrane sheet being formed through draining by direct contact (conduction) with a heated surface or by irradiation of the surface of the membrane sheet (radiation heat), or combination thereof. At the same time heat is applied, water is drained through pressure difference that exists on the opposite sides of the patterned filter. This can be accomplished by reduced pressure, increased pressure, or by pressing mechanically the membrane sheet with the heated surface.

In one aspect of the invention heat is applied in by contacting the nanofibrillar polysaccharide membrane with a heated surface optionally coated with a non-adhesive layer.

Heat may be applied to the membrane sheet being formed to raise its temperature to a range which is below the boiling point of the liquid to promote removal of the liquid in liquid state.

When the pressure difference is achieved by pressing the membrane sheet with a heated surface against the patterned filter, the final draining of the liquid out of the membrane sheet can be enhanced by placing an absorbent sheet against the free side of the patterned filter to absorb the drained liquid. Examples of suitable absorbents include absorbent pulp sheets, blotting papers and drying felts. Such absorbent sheets can be placed in layers against the free side of the patterned filter. Such an absorbent sheet or plurality of absorbent sheets removes liquid by absorption from the patterned membrane comprising nanofibrillar cellulose sheet being formed.

In one aspect of the invention the heated surface and/or non-adhesive layer is patterned and the inverse image of the pattern is transferred to the side of the membrane facing the heated surface when the heated surface is pressed in direct contact against the membrane.

In one aspect heat is applied to the nanofibrillar polysaccharide membrane from the heated surface through an optionally patterned layer interposed between the heated surface and the nanofibrillar polysaccharide membrane, such as a filter patterned or a structural layer to which the nanofibrillar polysaccharide membrane is to be laminated.

In one aspect the nanofibrillar polysaccharide dispersion is provided on a moving patterned filter as a continuous layer and a continuous patterned membrane is produced by transferring the continuous layer on the moving patterned filter through different processing steps, and the patterned membrane is separated from the patterned filter.

Certain grades of the nanofibrillar polysaccharides are especially hard to dry because of their water retention capacity and the drying may take considerably longer than with normal "native" grades. Nanofibrillar cellulose containing anionic groups are an example of nanofibrillar polysaccharide dispersions that are particularly difficult to dry. Cellulose obtained through N-oxyl mediated catalytic oxidation (e.g. through 2,2,6,6-tetramethyl-1-piperidine N-oxide) or carboxymethylated cellulose are specific examples of anionic nanofibrillar cellulose where the anionic charge is due to a dissociated carboxylic acid moiety. These anionic nanofibrillar cellulose grades are potential starting materials for preparing membranes, because high quality nanofibrillar polysaccharide dispersions are easy to manufacture from chemically modified pulp. In order to enhance drying of membranes comprising nanofibrillar anionic cellulose said cellulose can be pretreated by lowering the pH of the dispersion. In one aspect pH can be lowered by adding a suitable acid. This pretreatment reduces the water retention capacity of the anionic cellulose. In one aspect by lowering the pH of the nanofibrillar polysaccharide dispersion to below 3 pH units the drying time using the above-described methods can be reduced. Suitably an acid which is therapeutically compatible is used in case patterned membranes are prepared for medical use.

High aspect ratio of length facilitates maintaining the nanofibrils on the filter fabric. However, if the size of the polysaccharide nanofibrils is very small, they may flow through the filter fabric together with the liquid to be removed even if the smallest possible pore size of the filter fabric is used. According to one aspect of the invention, the flow of polysaccharide nanofibrils through the filter cloth is prevented by providing a first fibrous polysaccharide dispersion layer on the filter fabric and forming a fibril network by draining the liquid through the filter fabric that is impermeable to the fibrils of the first fibrous polysaccharide dispersion. This fibril network acts as an additional filter for the second nanofibrillar polysaccharide dispersion applied subsequently wherein the size of the fibrils in the second cellulose dispersion is smaller than that of the fibrils in the first fibrous polysaccharide dispersion. After the application of the second nanofibrillar polysaccharide dispersion the draining proceeds as with the fibrous polysaccharide dispersion applied in one step above.

The size of the fibrils of the second fibrous polysaccharide dispersion is selected such that compared with the pore size of the filter fabric they would penetrate through the fabric together with the liquid (filtrate) drained from the dispersion. The quantity of the second nanofibrillar polysaccharide dispersion may be larger than the quantity of the first nanofibrillar polysaccharide dispersion and, consequently, it may constitute the largest part of the weight of the dried membrane.

The patterned filter fabric is suitably used which has a pore size sufficiently small in relation to the fibril size to ensure efficient filtering of permeate from the nanofibrillar polysaccharides and while not allowing substantial transfer of nanofibrillar polysaccharides through the filter cloth. Suitably the pore size of filter fabric is in the micrometer range. Typically the mesh opening/porosity is from 0.1 to 50 micrometer, preferably 1 to 10 micrometer. Wire diameter of filter cloth is 1 to 200 micrometers, preferably 10-100 micrometers. The filter fabric may be made of a material which is preferably non-adherent to the filtered nanofibrillar polysaccharide membrane sheet, such as plastics and other synthetic polymers such as PET, polyamide and fluoropolymers. Another non-limiting example of a suitable fabric is tightly woven polyamide-6,6 fabric that are available in various pore sizes, which can be selected according to the selected particle size of the nanofibrillar cellulose.

The surface of the filter fabric may be modified such that it produces the selected pattern on the surface of the nanofibrillar polysaccharide membrane during the manufacturing process of the membrane.

The heated surface for providing heat into the nanofibrillar polysaccharide is suitably non-adherent to the filtered nanofibrillar polysaccharide membrane sheet. A metal plate coated with a repellent and heat-resistant coating, such as PTFE, can be used. In one aspect the heated surface can be patterned with an inverse pattern of the desired pattern to be created on the side of the nanofibrillar polysaccharide membrane. The pattern is formed when the heated surface is pressed against the membrane.

The inventive method above can be used for manufacturing separate individual membranes successively one by one in a sheet mold by applying the nanofibrillar polysaccharide dispersion on a filter fabric and performing successive work stages according to a predetermined sequence. Alternatively, the inventive method above can be used for manufacturing a continuous membrane in a continuous process by applying the nanofibrillar polysaccharide dispersion on a moving filter fabric which carries the membrane sheet being formed through successive work stages.

The starting concentration of the nanofibrillar polysaccharide dispersion that is applied on the filter fabric is usually not higher than 5w%, for example in the range of 0.5 - 5.0w%. This is usually the initial concentration of the nanofibrillar polysaccharide at the exit the manufacturing process where it is manufactured by disintegrating fibrous raw material. However, it is possible that the nanofibrillar polysaccharide dispersion is diluted with a liquid from the initial concentration (concentration of the product from the manufacturing process) to a suitable starting concentration to ensure that it is distributed evenly on the filter fabric to avoid variations in the membrane structure. Depending on the characteristic viscosity of the nanofibrillar polysaccharide grade, the starting concentration can be lower or higher, and it can vary between 0.1 and 10w%. Examples of suitable starting concentrations for the nanofibrillar polysaccharide dispersion according to the embodiments of the invention are 0.1, 0.2, 0.3, 0.4, 0.5, 0.6, 0.7, 0.8, 0.9, 1, 2, 3, 4, 5, 6, 7, 8, 9, and 10w%. Higher concentrations can be used for low-viscosity grades, which can be spread uniformly on the filter fabric despite their high concentration.

When water is the liquid to be drained, heat is applied to the nanofibrillar polysaccharide provided on the filter fabric preferably at the intensity that raises the temperature of the nanofibrillar polysaccharide at least to 70°C but below 100°C, for example in the range of 70-95°C. Contrary to what might be expected, raising the temperature above 100°C does not improve the drying result, because as long as the membrane sheet contains large amounts and water and the water is removed through pressure difference in the initial stages of drying, water must not be allowed to boil because this will have a detrimental effect on the membrane. When the membrane sheet is dry enough and no further water is extractable from the sheet by pressure difference, the residual water still bound to the finally formed fibril network of the sheet can be removed by evaporation. In this case temperature higher than 100°C can also be used.

However, it is possible that a filtration layer is used that retains the cellulose fibrils while allowing the liquid to pass, in the same purpose as the filter fabric, but will remain adhered to the membrane sheet and will form part of the membrane product. In this case the filtration layer can be made of a material that is adherent to the cellulose fibrils of the membrane sheet, and it can be for example made of cellulose fibers.

Auxiliary agents for enhancing the manufacturing process or improving or adjusting the properties of the membrane can be included in the nanofibrillar polysaccharide dispersion. Such auxiliary agents can be soluble in the liquid phase of the dispersion or solid. Auxiliary agents can be added already during the manufacturing of the nanofibrillar polysaccharide dispersion to the raw material or added to fibrous polysaccharide dispersion before applying it on the filter fabric. For cell therapy applications auxiliary agents may comprise agents supporting cell growth, adhesion.

To form a solid free-standing patterned membrane where fibrils are arranged in a network, liquid must be removed from the dispersion. Liquid can be removed from nanofibrillar polysaccharide by an illustrative method comprising one or two steps. In the first step liquid is drained by a pressure gradient between the two sides of the nanofibrillar polysaccharide dispersion/membrane.

In one aspect negative pressure is created on the filter side of nanofibrillar polysaccharide dispersion.

In another aspect increased pressure is used on the side of nanofibrillar polysaccharide dispersion which is opposite to the filter cloth, optionally together with negative pressure on the filter side of nanofibrillar polysaccharide dispersion as above.

In case a two-step method is used, in the second step heat is applied on the membrane while a pressure difference is maintained over the filter fabric, causing further drainage from the membrane sheet.

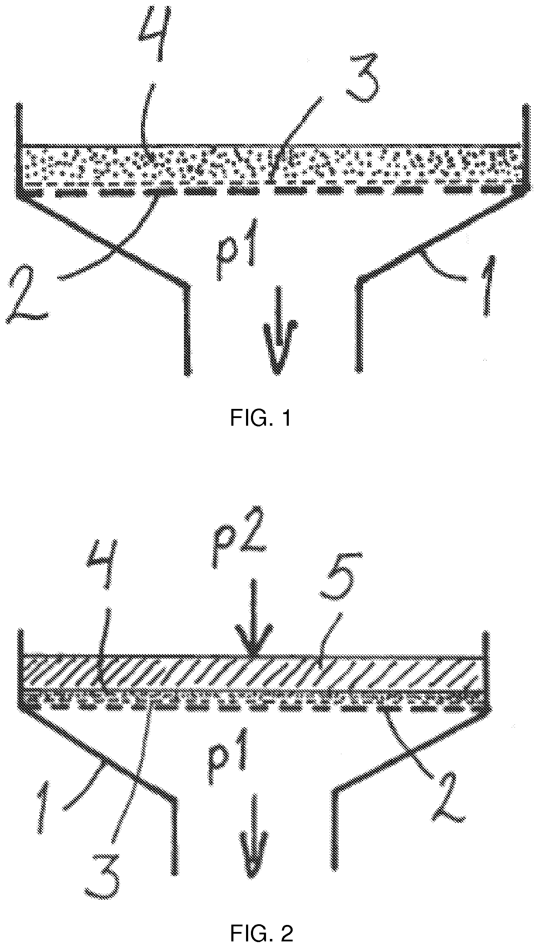

Figs. 1 and 2 show as embodiment of manufacturing the patterned membrane according to the invention wherein a modified laboratory sheet mold 1 is used. In the Fig. 1 and in other Figs. 2-4 illustrating the inventive method various elements are not drawn to scale. Aqueous nanofibrillar polysaccharide dispersion 4 is applied on top of a patterned filter fabric 3 which has holes in micrometer range and which has micro-scale topography comprising recesses and/or protrusions arranged in the inverse arrangement compared to the pattern to be formed on the membrane. The patterned filter fabric 3 is supported suitably by a wire 2 of the sheet mould 1. In the first step shown in Fig. 1 the dewatering from the polysaccharide dispersion 4 through the patterned filter fabric 3 and wire 2 is caused by reduced pressure p1 (vacuum) that is effective on the free side of the patterned filter fabric 3 (side not covered by the nanofibrillar polysaccharide dispersion 4). Thus, water flows through the patterned filter fabric and wire and the dry matter content of the polysaccharide dispersion 4 is gradually increased concurrently with the removal of water.

After a wet membrane sheet 4 is formed on the patterned filter fabric through dewatering and dewatering through the patterned filter fabric 3 has ceased, the second step shown in Fig. 2 may be initiated. The surface of a heated body 5 is placed on top of the membrane sheet 4 and the membrane sheet is pressed with its whole surface in contact with the body 5 against the patterned filter fabric 3 and the reduced pressure p1 (vacuum) is maintained. The pressure caused by the heated body 5 is designated p2 (arrow). The dewatering continues through the combined effect of the pressure p2 and the reduced pressure p1, which causes a pressure difference over the filter fabric and removal of more water from the membrane sheet through the filter fabric. Simultaneously as dewatering continues, the nanofibrillar polysaccharide membrane settles firmly against the patterned filter's micro structure and fibers fill the micro scale recesses of the patterned filter while the side of the membrane 4 which is against the body 5 remains smooth.

The surface of the body 5 transfers heat to the membrane sheet 4 which enhances dewatering because of the rise of the temperature of the membrane sheet 4 and especially temperature of the water contained in it. The temperature of the body 5 can be for example 90°C. The body 5 can be of metal. The contact surface of the metal body may optionally be coated with a thin coating that prevents adherence of the membrane sheet 4, for example PTFE, which is resistant to temperatures used in heating the membrane sheet 4. Optionally the body 5 and/or the coating is patterned such that the surface of the membrane 4 which is against the body 5 is patterned with an inverse pattern of the pattern on the body 5 and/or the coating. This enables manufacturing membranes having patterns on both sides. In the Figure 2, the body 5 is an unpatterned metal plate.

The body 5 is preferably preheated so that the temperature of the membrane sheet 4 starts to rise immediately after it has been placed against the membrane sheet 4. The body 5 is heated externally during the pressing so that the temperature is maintained at a constant level.

After the dewatering has proceeded to a suitable dry matter content, the membrane sheet 4, which is self-supporting membrane because of the formed cellulose fibril network, is detached from the filter fabric 3 and removed from the mold 2. The mold 2 can be used thereafter for the manufacture of the next membrane.

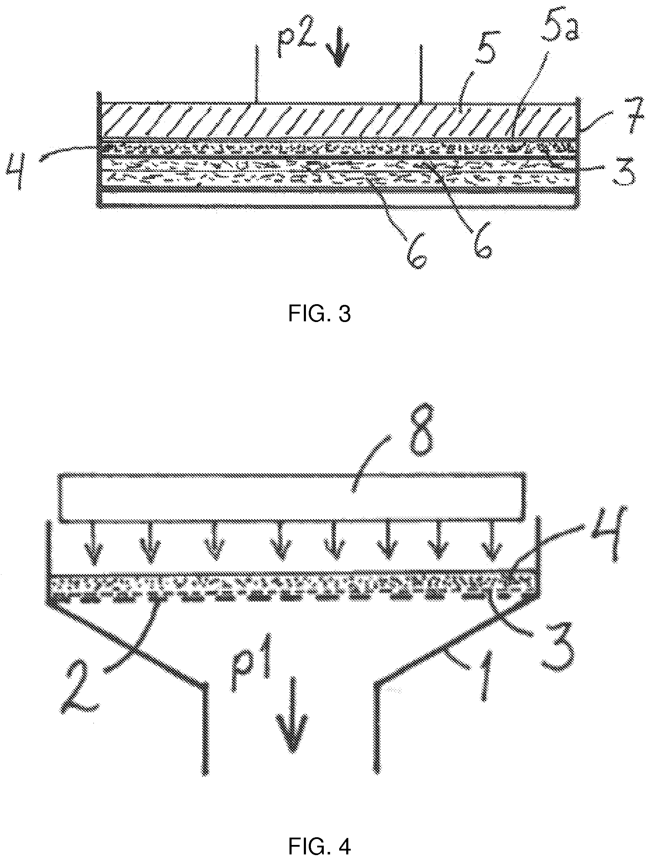

In the embodiment of Figs. 1 and 2, all steps are performed in the same sheet mold 2. Fig. 3 shows an embodiment where the dewatering from the dispersion 4 through the patterned filter fabric 3 and wire 2 was initially caused by reduced pressure p1 in conformity with Fig. 1. Fig. 3 shows a further step, where the wet membrane sheet 4 together with the patterned filter fabric 3 is removed from the sheet mold 1 and transferred to a press 7 where it is placed with the filter fabric on one or several absorbent sheets 6 so that the free surface of the patterned filter fabric 3 comes in contact with the surface of the absorbent sheet 6. The absorbent sheet 6 can be made of fibrous material and is capable of receiving water inside its volume. The sheet 6 can be absorbent pulp sheet, blotting paper or piece of drying felt. As shown by Fig. 3, the sheets 6 can be stacked to increase the water-receiving volume.

A heated body 5, which can have a similar structure and function as in Fig. 2, is placed on the free surface of the wet membrane sheet 4. Mechanical pressure p2 is applied to the membrane sheet 4 by means of the body 5. Dewatering is caused by the pressure difference effected by the mechanical pressure p2 only, and the water squeezed out of the membrane sheet 2 flows through the filter fabric 3 into the absorbent sheet 6 or absorbent sheets, where it is retained by the volume of the absorbent sheet(s) 6. The heat is transferred from the body 5 to the membrane sheet 4 as in the embodiment of Figs. 1 and 2. Below the absorbent sheet(s) 6 there can be a cold metal surface which is kept at a relatively low temperature so that a temperature gradient is created through the wet membrane sheet 4 and the absorbent sheet(s) 6 to urge water from the high temperature towards the lower temperature. The temperature of the metal surface can be adjusted for example below 25°C, preferably below 20°C. The non-adherent coating on the contact surface of the body 5 is designated 5a. After the dewatering has proceeded to a suitable dry matter content, the membrane sheet 4 and the filter fabric 3 are detached from the press 7 and the membrane sheet 4, which is self-supporting membrane because of the formed cellulose fibril network, is detached from the press filter fabric 3. The filter fabric 3 can next be used in the sheet mold 1 for the formation of a new membrane sheet 4. The absorbent sheet or sheets 6 is/are detached from the press 7, dried, and they may be reused in the press 7.

In one embodiment the body 5 and/or non-adherent coating 5a is provided as having a pattern comprising micro-scale recesses and/or protrusions. Said pattern can be used to create the inverse pattern on the otherwise smooth side of the membrane when pressure is applied and the membrane is formed.

The surface of the bulk layer (i.e. the opposite side of the patterned side) can be also modified by using pattern transfer with pattern calendering in a continuous web process or with static embossing press in a non-continuous process.

In the embodiment of Fig. 3, the first step (dewatering by vacuum) takes less than 60 s when the target grammage of the membrane is 20 gram per square meter. The second step (pressing + heating) takes less than 5 minutes. The total preparation time starting from the nanofibrillar polysaccharide dispersion and ending in a dry membrane is less than 10 minutes, whereas in conventional methods the preparation time can be several hours.

Fig. 4 shows an embodiment where the first step was performed as in Fig. 1, by reduced pressure p1 (vacuum). The heat applied on the opposite side of the membrane sheet 4 being formed is not accomplished by contact (conduction) with the heated surface 5 as in Figs. 2 and 3, but by irradiation of the free surface of the membrane sheet (radiation heat) by an IR heating device 8 that is placed at a distance from the membrane sheet 4. Mechanical pressure is not applied, but the water is drained from the membrane sheet 4 through the filter fabric 3 by the effect of pressure difference caused by the reduced pressure p1 only. The micro scale pattern is formed on the side of the membrane which is in contact with the filter fabric 3.

To create the patterned surface, the filter membrane is selected or modified such that it has a surface having patterns that produce on the nanofibrillar polysaccharide membrane the selected patterned surface comprising recesses and/or protrusions when pressed against the nanofibrillar polysaccharide membrane during drying. The pattern on the filter cloth is inverse compared to the pattern on the filter membrane. As is obvious to a person skilled in the art, any pattern can be created on the nanofibrillar polysaccharide membrane by using the method according to the embodiments of the present invention. For example, the inverse pattern of the pattern of a typical filter cloth can be created on the nanofibrillar polysaccharide membrane. Alternatively, the inverse pattern of the desired pattern can be made on the filter cloth using methods known in the art.

Compared with dewatering of nanofibrillar polysaccharide dispersions where the polysaccharide is native cellulose, dewatering of nanofibrillar polysaccharide dispersions where the polysaccharide is anionic cellulose is even more time-consuming because water is bound very strongly to the cellulose. Nanofibrillar cellulose containing anionic groups can be for example chemically modified cellulose that contains carboxyl groups as a result of the modification. Cellulose obtained through N-oxyl mediated catalytic oxidation (e.g. through 2,2,6,6-tetramethyl-1-piperidine N-oxide, known by abbreviation 'TEMPO") or carboxymethylated cellulose are examples of anionic nanofibrillar cellulose where the anionic charge is due to a dissociated carboxylic acid moiety. The total drying time is expected be many times the total drying time with nanofibrillar cellulose where the cellulose is unmodified, mainly due to the higher water retention capacity and higher viscosity of the anionic nanofibrillar cellulose. For example, dewatering unmodified nanofibrillar cellulose in the first step when the target is a 20 gram per square meter membrane takes less than 60s (time from starting the vacuum until no visible water is seen on the membrane sheet), whereas dewatering of a anionic nanofibrillar cellulose for a membrane with the same target grammage in similar conditions can take even 60 to 120 minutes.

The dewatering properties of these anionic nanofibrillar cellulose grades can be considerably improved by pre-treating the nanofibrillar polysaccharide dispersion by an acid. When the nanofibrillar cellulose contains anionic groups that act as bases (acid moieties in dissociated from), as is the case with oxidized cellulose and carboxy methylated cellulose, lowering the pH with acid will converts these groups into an undissociated form, the electrostatic repulsion between the fibrils is no more effective, and the water-fibril interaction is changed in a way that favours dewatering of the dispersion (water retention capacity of the dispersion is reduced). The pH of the anionic nanofibrillar cellulose dispersion is lowered below 4, preferably below 3, to improve the dewatering properties.

Anionic nanofibrillar cellulose dispersion which was obtained from 'TEMPO" oxidized pulp needed a dewatering time under vacuum of roughly 100 min at original (unadjusted) pH, when the target grammage of the membrane was 20 gram per square meter. When the pH of the dispersion was lowered to 2 with HCl before dewatering, the dewatering time in the same conditions was about 30 seconds, that is, the time was reduced to 0,5% of the original. When pH is lowered, the dispersion becomes visibly aggregated (fibril flocks are formed), which is believed to be one reason for faster dewatering because water flows more easily between the aggregates. The membrane sheets formed in the first step by dewatering the dispersion with lowered pH can be dried to its final dryness in the second step. The tendency of the membranes to tear during the final stages of the drying, which is probably due to the initially aggregated structure of the dispersion at low pH, can be eliminated by interrupting the drying. The membrane sheet is then allowed to lie free and detached from any supporting structure (such as filter fabric) to relieve the stresses. Thereafter the drying can be continued. The final stages of the drying can be performed between two absorbent sheets (for example blotting papers) at a temperature above 100°C, for example at 105°C, to remove remaining moisture.

If the fibril size of the anionic nanofibrillar cellulose is too small with regard to the filtration capacity of the filter fabric (cutoff size), which often is the case with nanofibrillar cellulose made from oxidized pulp, an auxiliary filter layer can first be formed of fibrous polysaccharide dispersion with larger fibril size on the same principle as explained above, before the pre-treated nanofibrillar polysaccharide dispersion is added. The auxiliary filter layer can be made for example of chemically unmodifed (native) fibrous polysaccharide dispersion, such as cellulose, where the fibril size is larger.

When nanofibrillar polysaccharide dispersions are applied to the filter fabric, they can be applied by pouring, or some other application methods for making initially a uniform layer of the dispersion with minimal thickness variations. Dispersions can for example be sprayed on the filter fabric. If necessary, dispersion may be diluted with water to decrease the viscosity and improve the uniform spreading of the dispersion.

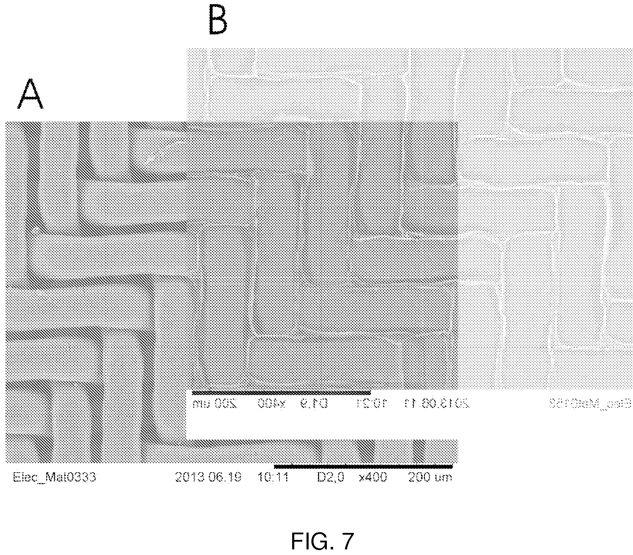

The resulting patterned nanofibrillar polysaccharide membranes can be manufactured in various thicknesses depending on the desired characteristics of the membrane. Thin membranes with uniform grammage distribution (small grammage variation over the area of the membrane) can be prepared. The selected patterning has an effect on the mechanical properties of the resulting membrane and in general a more rigid structure is obtained when the membrane is patterned, as compared to unpatterned membrane of the same thickness. The total thickness of the membranes is preferably no higher than 150 $\mu$m. If a freestanding membrane is prepared, the thickness is preferably in the range of 10 to 100 $\mu$m and still more preferably 30 to 70 $\mu$m to confer sufficient strength, whereas when forming a membrane layer in a membrane product (either adhered to the filter layer or laminated separately to a support) its thickness can be smaller, such as in the range of 5 to 40 $\mu$m. However, these numerical values should not be regarded as restrictive. Non-limiting examples of membrane thicknesses according to the embodiments of the invention are 1, 2, 3, 4, 5, 6, 7, 8, 9, 10, 15, 20, 25, 30, 35, 40, 45, 50, 55, 60, 65, 70, 75, 80, 85, 90, 95, 100, 110, 115, 120, 125, 130, 135, 140, 145 and 150 $\mu$m. Along the cross section of the patterned membrane (perpendicular to the plane of the membrane) two structural layers can be named in the continuous structure: a bulk layer and the patterned layer. The bulk layer essentially comprises the major volume of the patterned membrane, and the patterned layer corresponds to the portion of the patterned membrane which comprises the micro scale topography. As is seen e.g. in Fig. 7 the thickness of the patterned layer is determined at least partly by the properties of the filter, i.e. how deep inside the filter the nanofibrillar polysaccharide may penetrate.

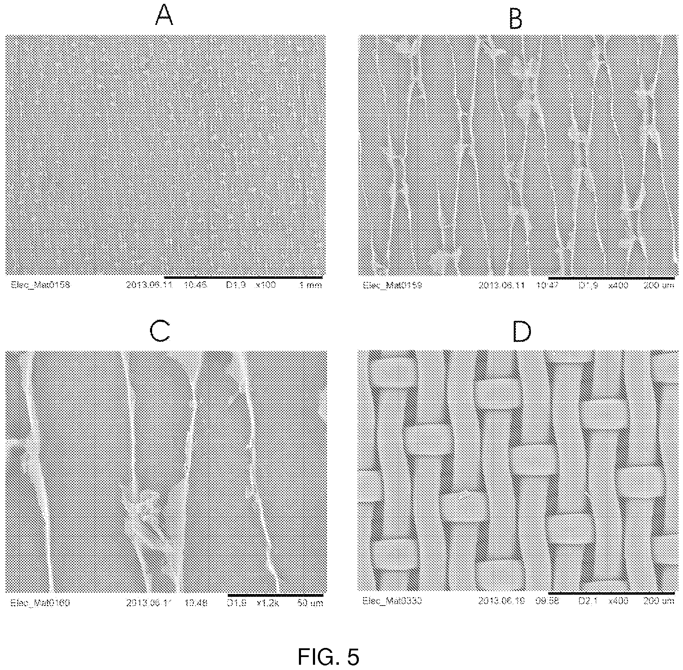

Preferably, the patterned nanofibrillar polysaccharide membranes are dry and have a residual moisture content of < 10 w%. Preferably, the residual moisture content is about 9, 8, 7, 6, 5, 4, 3, 2, or 1 w%. Visually membranes dried to this dryness are translucent and rigid sheet-like membranes. When the membranes are prepared using an unpatterned heated surface, the upper side of the membranes facing the heated surface is very smooth with minimal surface roughness. The bottom part has the distinct surface structure arising from the morphology of the filter fabric, see Figures 5-8.

In the present application liquid removal is described when water is the dispersing medium that is to be removed from the nanofibrillar polysaccharide dispersion. The operations can be performed analogically when other liquid than water is the dispersing medium.

## Structure of the patterned membrane

The patterned membrane is a continuous structure comprising nanofibrillar polysaccharides, such as nanofibrillar cellulose. In an embodiment of the invention two layers can be seen in the patterned membrane: a bulk layer and a patterned layer. It should be understood that the above reference to the bulk layer and the patterned layer is not intended to mean that said layers are physically separate layers as such, but said terms are used solely for the ease of description of the invention and, consequently, it means the structurally different parts of the patterned membrane, i.e. the part of the continuous membrane which is patterned, and the part which is not patterned.

## Device

Aspects of the present invention are related to developing a micro-scale patterned nanofibrillar polysaccharide membrane as a seeding scaffold or a device to directly apply therapeutically useful cells, such as stem cells, on the wound site to improve skin wound healing, closure and/or to reduce inflammation on the wound site. The invention allows controlled delivery of therapeutic cells to the treatment site by administering them by using a vehicle comprising patterned nanofibrillar polysaccharide membrane and the extracellular matrix secreted by the cells.

In one aspect the device has at least one side of its membrane coated or chemically bonded with an agent which enhances cell adhesion to the membrane. These agents include all kind of extracellular matrix proteins such as laminin, fibronectin, vitronectin, type I collagen, type II collagen, or type IV collagen or combinations or fractions thereof or complex mixtures. In another aspect, when the membrane is patterned on both sides, the different sides of the membrane may be coated or chemically bonded with same or different agents. Suitably the agent(s) is selected from the group consisting of proteins, peptides, carbohydrates, lipids, nucleic acids and fragments thereof, anti-viral compounds, anti-inflammatory compounds, antibiotic compounds such as antifungal and antibacterial compounds, cell differentiating agents, analgesics, contrast agents for medical diagnostic imaging, enzymes, cytokines, anaesthetics, antihistamines, agents that act on the immune system, immunostimulatory agents, hemostatic agents, hormones, angiogenic or anti-angiogenic agents, neurotransmitters, therapeutic oligonucleotides, viral particles, vectors, growth factors, retinoids, cell adhesion factors, osteogenic factors, antibodies, antigens, peptides, cells and their derivatives including acellular matrix.

In one aspect the device may additionally comprise one or more layers extending over at least part of the device, such as the central or peripheral area of the device, the one or more layers being selected from a support layer, a backing layer, a moisture retaining layer, a moisture absorbing layer, a moisture barrier layer, a gas barrier layer, an odour absorbing layer, a drug-containing layer, an adhesive layer and/or a mucoadhesive layer.

In one aspect the device comprises aqueous medium selected from the group consisting of water, sterile water, purified water, physiological saline, a physiological buffer, a culture medium, nutritional agents, and/or a bioactive agent, or combinations thereof.

In one aspect the therapeutically useful cells comprise autologous cells, allogeneic cells, stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, cord blood cells, embryonic stem cells, induced pluripotent cells, placental cells, bone marrow derived cells, immune system cells, hematopoietic cells, dendritic cells, hair follicle cells, chondrocytes, hybridoma cells, and combinations thereof.

In one aspect the device comprises cells useful for wound healing, preferably mesenchymal stem cells, adipose-derived stem cells or bone-marrow derived stem cells.

## Wound treatment

Aspects of the present invention relate to use of the device according to an embodiment of the invention for wound treating. When the nanofibrillar polysaccharide membrane is used in the device it is preferably obtained from non-animal material such as plants. For biomedical applications plant-derived material is preferred. In one aspect the cells used in the device may be of human origin. In another aspect the cells can be of non-human origin. The cells can be autologous or heterologous.

In one aspect hASCs are obtained by lipoaspiration prior to treatment either from the subject to be treated (when autologous cells can be isolated) or from donors (Escobedo-Lucea et al. A Xenogeneic-Free Protocol for Isolation and Expansion of Human Adipose Stem Cells for Clinical Uses, Plos One, July 9, 2013). According to this embodiment the isolated hASCs are cultured on the NFC culturing matrix until a desired cell density is reached.

As used herein, the term "wound" is used to refer broadly to injuries located in all layers of skin, epidermis, dermis and subcutaneous tissue, initiated in different ways and with varying characteristics.

The term "kit" refers to a combination of articles or containers that facilitate a method, assay, or manipulation of the compositions according to the embodiments of the invention. Kits can optionally contain instructions describing how to use the kit (e.g., instructions describing the methods of the invention), cartridges, mixing stations, chemical reants, as well as other components. Kit components may be packaged together in one container (e.g., box, wrapping, and the like) for shipment, storage, or use, or may be packaged in two or more containers.

Even though any cell can be cultured on the patterned nanofibrillar polysaccharide membrane, for wound treatment suitable cells are autologous or non-autologous mammalian adipose derived stem cells.

The cells cultured using the present polysaccharide membrane matrix can be transported without need for freezing the cells before or during transportation. In one aspect the cultured cells can be transported to the site of treatment directly after culturing them e.g. at +37°C without additional steps. The cultured stem cell lines can be also genetically engineered to produce into the culture system a selected protein, such as a growth factor, immunomodulatory protein or other agent improving wound healing.

In another aspect the nanofibrillar polysaccharide membrane is coated with at least one side with laminin to enhance cell adhesion to the membrane.

The following examples are given solely for the purpose of illustrating various aspects of the invention.

## EXAMPLES

### Materials

Nanofibrillar cellulose

The nanofibers were isolated from bleached birch pulp with Masuko Sangyo's Supermasscolloider with 9 passes through the grinding stones. The end product had the following characteristics:

- Concentration 2.0 weight%

- Translucent or opaque, turbidity 150 AU

- Sligthly anionic surface charge, -2 mV

- Fiber diameter 7 nm nanofibers + 20-50nm fibril bundles, length several micrometers.

- Number of un-fibrillated particles 200 (particles / mg), FiberLab

- Carbohydrate composition: 72.8% Glucose, 25.6% Xylose, 1.4% Mannose

- Zero shear viscosity of 0.5 wt% sample 8 000 Pa s and yield stress 5 Pa.

- Zero shear viscosity of 1.0 wt% sample 30 000 Pa s and yield stress 20 Pa.

- Storage modulus of 0.5 wt% sample G' = 10 Pa

**EXAMPLE 1**

**Nanofibrillar polysaccharide membrane preparation**

A two-stage method for preparation of NFC membranes was used. In the first stage a wet NFC membrane was formed using a modified laboratory sheet mold. A filter cloth, either 1 micrometer or 10 micrometer porosity were utilized. The filter fabrics were Sefar Petex 07-10/2 and Sefar Petex 07-1/2, their wire diameters were 47 and 34 micrometers, respectively. Firstly, the filter cloth was placed on top of the sheet mold wire and the NFC dispersion was poured on it. Consistency of NFC dispersion was 2 g/l, but it may be necessary to change it according to the situation and properties of NFC. Sheet mold vacuum was used to remove water from the NFC dispersion.

When water was no longer removed from the forming NFC-membrane a teflon coated metal plate was placed on top of the NFC-membrane, so that the membrane was between the metal plate and polyamide fabric. Cellulose blotters were placed under the polyamide fabric.

In the second stage the blotter/polyamide/NFC-membrane/metal plate package was removed from the sheet mold and taken to a hydraulic press. Upper plate of the press was heated to 90°C, and the teflon coated metal plate was placed against it. The pressing was started and continued for a few minutes. During this period water that was not removed during first stage from the membrane transferred to blotters, and strong internal bonding was formed within the NFC membrane so that it could easily be removed from the polyamide fabric. Simultaneously, the negative image of the surface morphology of the filter fabric is transferred to the formed NFC membrane.

**NFC membrane properties**

In this study, 60 g/m2 NFC membranes were made with approximately 60 micrometer thickness. Dry density of the membranes was 1.4-1.5 g/cm3. After the production, the membranes were dry (1-5w% residual moisture), translucent and rigid sheet like materials. The upper side of the membranes was very smooth with minimal surface roughness. The bottom part had the distinct surface structure arising from the morphology of the filter fabric, see Figures 5-8.

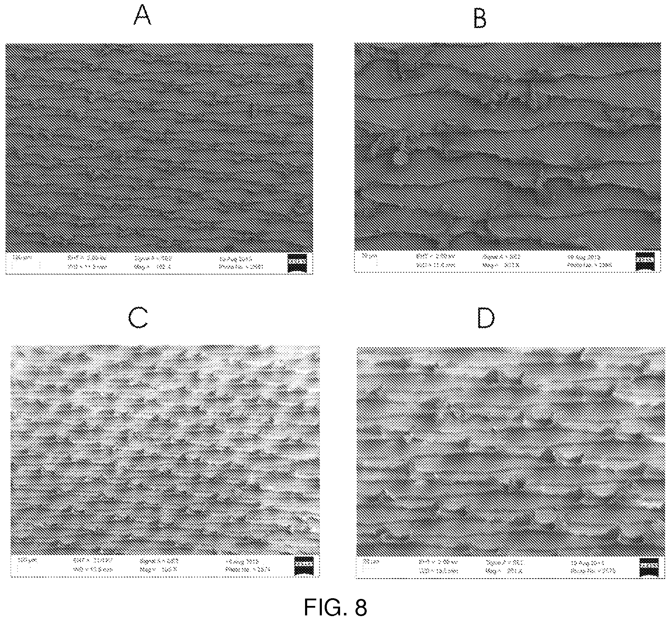

In Figure 5, the surface structure of the NFC membrane made with 1 micrometer filter cloth is presented. A continuous protruding pattern is formed: the structure is composed of long closed diamond shaped well structure where the diagonal length is close to 300 micrometers. Between the longer shapes, also shorter rectangular well shapes can be seen. The SEM image of the corresponding filter cloth reveals that the surface structure has not been directly copied; the well shapes are stretch to diamond shapes. The stretching is caused by shrinkage upon drying of the wet NFC membrane during the drying stage 2. This behavior is well known e.g. in paper manufacturing. Controlled drying shrinkage can be used to adjust the dimensions of the shape after drying is completed. The diamond-type of shape is formed when part of the material has a firm contact with the fabric filament and the latter part has much less contact with the filament surface. This leads to local differences in drying shrinkage and the rate of drying resulting in a non-rectangular shape. This phenomenon can be controlled with the fabric waving pattern, structure and filament type. The stretched shaped can be relaxed by moisturizing the membranes: the relaxed rectangular shape can be seen from the SEM images related to cell culture experiments, see Figure 8. The height of the protruding wall structure can be roughly estimated from the SEM images, see Figure 8. For the membrane made with 1 micrometer filter, the height distribution of the protruding parts is broad; the lowest parts are only couple of micrometers while the highest part arise to 20-40 micrometers from the plain.

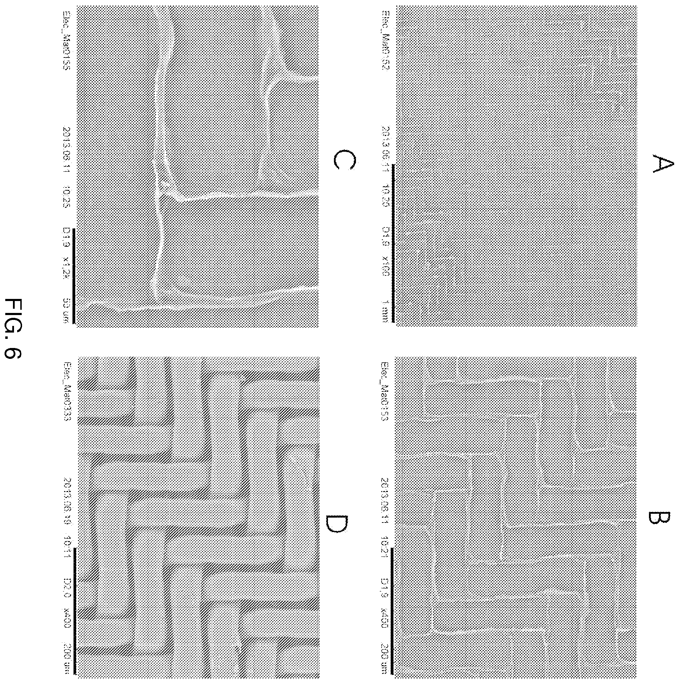

In Figure 6, the surface structure of the NFC membrane made with a 10 $\mu$m filter cloth is presented, as well as the corresponding SEM image of the filter. Although the yarn diameter (20 micrometer) is close to the size in 1 micrometer filter, the corresponding NFC membrane looks remarkably different compared to Figure 5. It seems that in the 10 micrometer filter cloth, the woven texture prevents the shrinkage during the drying stage and the surface structure of NFC membrane closely resembles the negative image of the filter, see Figure 7. The closed well structure is nearly rectangular (20 x 180 micrometers). The height of the protruding wall structure is difficult to estimate precisely from the SEM images, but could be around 5 to 10 micrometers.

## EXAMPLE 2

### Wound healing treatment

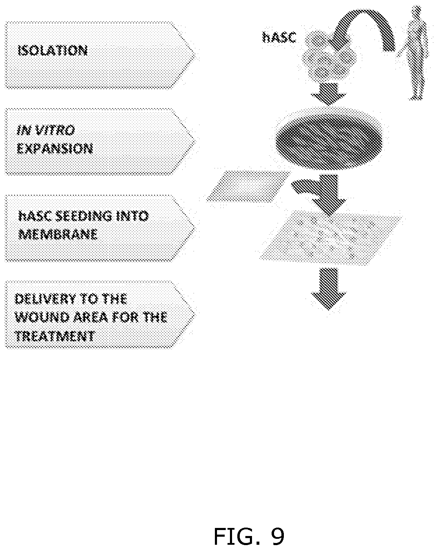

The scheme for the wound treatment is shown in Figure 9. Adipose mesenchymal stem cells (hASC), are seeded over the nanofibrillar cellulose membrane and cultured *in vitro* for one week before the delivery to the wound area. Isolation and culture conditions of the cells before their seeding over the membrane are developed following the protocol established by (Escobedo-Lucea et al, Plos One 2013).

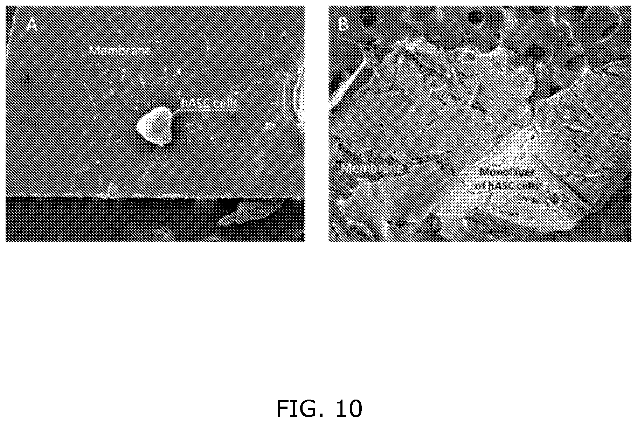

The nanofibrillar cellulose membrane has 2 different sides with 2 different properties, smooth and rough. hASC cells are seeded on the rough side of the membrane, given the fact that the mechanical adhesion is clearly better (see Figure 10). No chemical adhesion is needed at this point. In addition of that, the nanofibrillar cellulose membrane can be coated with any ECM derivative which may improve the adhesion of the hASC to the surface, but can be used without any coating as well.

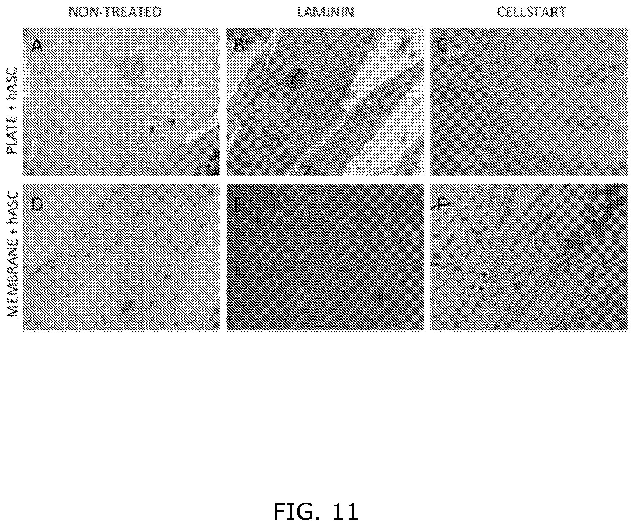

The safety of the nanofibrillar cellulose membrane for the cells has been checked (in all the cases) through different assays. After 7 days in culture, no morphological ultrastructural alterations were detected in the cells through Transmission Electron Microscopy (TEM) assays (Figure 11). Mesenchymal stem cell markers continue maintaining their characteristic levels when analyzed by QRT-PCR (Figure 12).

Cell dead is not increased after the culture of hASC over the membrane in any case.

Concerning *in vitro* immunomodulatory properties of hASC cytokine array studies were performed to check any alteration or difference between mesenchymal stem cells cultured over the nanocellulose membrane coated or non coated or in the traditional way over plastic. The cytokine expression and release does not seem to be compromised after the culture over the nanocellulose membrane (Figure 13 and Table 1). Cytokine expression ensures that everything is in function and no rejection is ongoing.

**Table 1.** Identification of cytokines released from cells cultured over plastic and membrane with the different coatings. The table shows those cytokines that are expressed with a relative pixel density higher than 5 %.

|  | **Plate** | **Membrane** |
| --- | --- | --- |
| Medium without human serum | SerpinE1 | SerpinE1 |
| Proteins secreted by cells (medium without HS) | MIF SerpinE1 | Il-1ra MIF Serpin E1 |
| Proteins secreted by LM + cells (medium without HS) | Groα il-1ra MIF SerpinE1 | Il-1ra MIF SerpinE1 |
| Proteins secreted by CS + cells (medium without HS) | Groα il-1ra IL-8 MIF SerpinE1 | MIF SerpinE1 |

Concerning *in vivo* wound repairing assays, the recovering of the wound area after the membrane with the cells treatment has been performed using the validated wound healing NUDE mice model described by Geer et al 2007.

After 5 and 10 days of cell membrane treatment, the animals were sacrified and anatomopathology studies were performed. The animals treated with the nanofibrillar membrane with cells, showed better healing prognostic as well as faster recovering as demonstrated in Figure 14.

### Materials and Methods

### Isolation and culture of hASC cells over membrane

Adipose mesenchymal stem cells were isolated and cultured using the protocol previously established by Escobedo-Lucea et al. 2013, with brief modifications in the case of the coated membrane. For the coating the membrane was cut under aseptic and sterile conditions inside the culture hood to have the desired area for cover the wound. Membrane was coated with 5 and 10 μg/ml of human laminin and CS respectively for 1 hour. After washing, cells were seeded in culture media and they were cultured in the incubator at 37°C, 95% of humidity and 5%CO2, for 1 week until the treatment or in vitro analysis. Culture media was changed every other day.

### Scanning electron microscopy

The cultures were immersion-fixed in 2.5% glutaraldehyde for 1 hour. Then postfixed in 1% osmium for 1 hour, dehydrated, critical point dried, sputter-coated, and analyzed under the scanning electron microscope (S-4100, Hitachi, Japan).

**Transmission electron microscopy**

For fine ultrastructural analysis, cells were cultured in chamber slides and then serially washed in a 0.1 M phosphate buffer (PB; pH 7.4) solution, prior to their fixation for Transmission Electron Microscopy (TEM). Fixation was performed in 3% glutaraldehyde solution in PB for 30 minutes at 37°C and postfixed in 2% $OsO_4$ in PB. Dehydration was achieved by a graded series of ethanol solutions and a final rinse with propylene oxide (Lab Baker, Deventry, Holland). Finally, plates were embedded in araldite (Durkupan, Fluka) overnight. Following polymerization, embedded samples were detached from the chamber slide and glued to Araldite blocks. Serial semi-thin (1.5 $\mu$m) sections were cut with an Ultracut UC-6 (Leica, Heidelberg, Germany), mounted onto slides and finally stained with 1% toluidine blue. Ultrathin (0.07 $\mu$m) sections were prepared with the Ultracut and stained with lead citrate. Photomicrographs were obtained under a transmission electron microscope (FEI Tecnai Spirit G2), using a digital camera (Morada, Soft Imaging System, Olympus).

**RNA preparation and QRT-PCR**

Total RNA was prepared from cells using RNeasy mini kit (Quiagen, Gilden; no. 74104). To eliminate contaminating genomic DNA, the initial RNA pellet was incubated with deoxyribonuclease (DNase) I (2 to 4 U/$\mu$L; Qiagen, Carlsbad; no. 79254) for 15 min at room temperature in the buffer su supplied by the manufacturer. RT-PCR and primer sequences were as described in Escobedo-Lucea et al 2013, Plos One). They were designed using Primer3 software and synthesized by Sigma-Aldrich. For each experiment, controls were performed in which reverse transcriptase was omitted from the cDNA reaction mixture and template DNA was omitted from the PCR mixture. For quantitative real-time PCR (QRT-PCR), 5 $\mu$g RNA was converted into cDNA, and a series of diluted samples were used for 40-cycle PCR in Light Cycler 480 SYBR Green I Master (Kit no. 04707516001) in a Lightcycler 480 (Roche Diagnostics, Mannheim) instrument. Reactions (20 $\mu$L total) contained 1 $\mu$L cDNA, 10 $\mu$M each primer, and 4 $\mu$M probe and were run using the default Lightcycler 480 program. To generate a standard curve for comparison of mRNA levels in different samples, multiple dilutions of the control cDNA sample, spanning at least 3 orders of magnitude, were prepared. The equation describing the plot of threshold cycle, Ct, versus log concentration was used to determine relative amounts of mRNA in experimental samples. Using the optimized conditions and threshold values, individual samples were analyzed in triplicate using the probe of interest and an internal control expected to be unchanged between samples. Three different internal controls were used: glyceraldehyde-3-phosphate-dehydrogenase (Gapdh), $\beta$-2 microglobulin, and $\beta$-actin. From the Ct values, the relative transcript concentration was calculated and normalized to that of the internal control. The maximum expression data point was adjusted to 100. Data are shown for samples normalized to Gapdh, but results were comparable when analysis was performed using either $\beta$-2 microglobulin alone or a combination of all 3 controls.

**Cytokine array**

The membranes were incubated with Streptavidin-HRP for 30 min. The array was revealed by adding Chemi Reagent Mix for 1 min. The excess of reagent was taken out and sealed. The membranes were placed with their identification in an autoradiography film cassette and exposed to Xray film from 1 to 10 minutes. The location and identity of controls, references and candidate cytokines are listed by the provider in the instructions.

To make the comparison between the conditions, pixel densities on developed X-ray film were collected and analyzed using a transmission- mode scanner and image analysis software (Image J). A template was created to analyze pixel density in each spot of the array. The average signal (pixel density) was determined using the pair of duplicate spots representing each cytokine taking into the account the signal from the clear area or negative control spots as a background. An averaged background signal from each spot was subtracted.

**Animal surgery**

Swiss nu/nu nude mice were purchased from Charles River (France) and housed in a facility maintained by the Centro de Investigacion Principe Felipe (CIPF) in Valencia, Spain with ethical permission number 12-02-38. We have them through our collaboration between Helsinki and Valencia agreement. For all experiments, male animals, 7-8 weeks of age were used. The experiments were approved from the CIPF Institutional Animal Care Committee. All procedures were performed with aseptic technique and all materials were sterile. Surgical procedures were performed in a biological safety cabinet and animals were housed in filter-topped cages in a laminar flow cage isolator. For the experiments, mice were placed in a gas chamber filled with isoflurane (IsoFlo; Abbott Laboratories, North Chicago, IL) until they reached the desired level of anesthesia. The pinch test was used and breathing rates were monitored to determine the appropriate level of anesthesia. The mice were then removed from the chamber and masked with isoflurane gas throughout the entire procedure. After washing the dorsum of the mouse with ethanol, we created two full-thickness wound (including the panniculus carnosus) 1 cm2 above the shoulder of the mouse, one of them was used as control and the other for treatment. Next, wound healing treatments were placed onto the wound and secured with a 6-0 Vicryl (Ethicon/Johnson & Johnson, Somerville, NJ) stitch at each corner. A piece of polyurethane occlusive dressing (Tegaderm; 3M, St. Paul, MN) was applied over the dressing. A trimmed 3M sports Band-Aid was placed over the dressing and sutured into place with a running 6-0 Vicryl stitch. Waterproof adhesive tape (Johnson & Johnson, Skillman, NJ) was then used to firmly wrap the graft and dressing into place. The wound area was hidrated using the recommendations described by Geer et al (2007).

**Histology**

Tissue morphology was assessed by standard hematoxylin and eosin staining and Masson's trichrome staining of paraffin-embedded tissue sections. For paraffin, excised skin equivalents were fixed in 10% buffered formalin (Fisher Scientific) for 2 h at room temperature followed by dehydration with ethanol-xylene washes. Tissues were embedded in paraffin after overnight infiltration at 60°C.

**Claims**

1. A device comprising
   a membrane comprising nanofibrillar polysaccharide arranged in a continuous arrangement, at least one side of the membrane comprising at least one patterned area comprising micro-scale recesses and/or protrusions, wherein the nanofibrillar polysaccharide comprises plant-derived nanofibrillar cellulose and wherein the patterned area comprises a repeating pattern of units, wherein at least one dimension of a unit is from 1 $\mu$m to 500 $\mu$m along the plane of the membrane, and
   therapeutically useful cells on the patterned area of the membrane.

2. The device according to claim 1, wherein the number average thickness of the patterned area is 100 nm - 100 $\mu$m, preferably 200 nm - 10 $\mu$m, and most preferably 1 - 10 $\mu$m.

3. The device according to any one of claims 1-2, wherein the patterned area comprises a repeating pattern of units interconnected with a common wall having a width from 10 nm - 10 $\mu$m, preferably 100 nm - 1 $\mu$m, most preferably 200 nm - 1 $\mu$m.

4. The device according to any one of claims 1-3, wherein the membrane has a thickness of 1 - 300 $\mu$m, preferably 10 - 100 $\mu$m, most preferably 20 - 60 $\mu$m.

5. The device according to any one of claims 1-4, wherein the membrane comprises 90 - 100 % by dry weight of nanofibrillar polysaccharide, preferably 95 - 100 % by dry weight of nanofibrillar polysaccharide, more preferably 99 - 100 % by dry weight of nanofibrillar polysaccharide.

6. The device according to any one of claims 1-5, wherein the nanofibrillar polysaccharide comprises a derivative of plant-derived cellulose.

7. The device according to any one of claims 1-6, wherein the nanofibrillar polysaccharide is mechanically disintegrated.

8. The device according to any one of claims 1-7, wherein the nanofibrillar polysaccharide comprises polysaccharide nanofibrils and/or nanofibril bundles having a number average diameter between 1 and 500 nm, preferably between 2 and 200 nm.

9. The device according to any one of claims 1-8, wherein the micro-scale recesses and/or protrusions have dimensions allowing the cells to accommodate the recesses of the membrane and/or allowing the cells to attach essentially on the protrusions of the membrane.

10. The device according to any one of claims 1-9, comprising aqueous medium absorbed inside the membrane, wherein the aqueous medium comprises water, sterile water, purified water, physiological saline, a physiological buffer, a culture medium, nutritional agents, and/or a bioactive agent, or combinations thereof, wherein said bioactive agent is selected from the group consisting of proteins, peptides, carbohydrates, lipids, nucleic acids and fragments thereof, anti-viral compounds, anti-inflammatory compounds, antibiotic compounds such as antifungal and antibacterial compounds, cell differentiating agents, analgesics, contrast agents for medical diagnostic imaging, enzymes, cytokines, anaesthetics, antihistamines, agents that act on the immune system, immunostimulatory agents, hemostatic agents, hormones, angiogenic or anti-angiogenic agents, neurotransmitters, therapeutic oligonucleotides, viral particles, vectors, growth factors, retinoids, cell adhesion factors, osteogenic factors, antibodies, antigens, peptides, cells and their derivatives including acellular matrix.

11. The device according to any one of claims 1-10, wherein at least part of the at least one side of the membrane is coated or chemically bonded with an agent for enhancing cell adhesion selected from the group consisting of all kinds of extracellular matrix proteins such as laminin, fibronectin, vitronectin, type I collagen, type II collagen, and type IV collagen and/or combinations or fractions thereof or complex mixtures.

12. The device according to any one of claims 1-11 additionally comprising one or more layers extending over at least part of the biomedical device, such as the central or peripheral area of the device, the one or more layers being selected from a support layer, a backing layer, a moisture retaining layer, a moisture absorbing layer, a moisture barrier layer, a gas barrier layer, an odour absorbing layer, a drug-containing layer, an adhesive layer and/or a mucoadhesive layer.

13. The device according to any one of claims 1-12, wherein the cells comprise autologous cells, allogeneic cells, stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, cord blood cells, embryonic stem cells, induced pluripotent cells, placental cells, bone marrow derived cells, immune system cells, hematopoietic cells, dendritic cells, hair follicle cells, chondrocytes, hybridoma cells, and/or combinations thereof.

14. The device according to any one of claims 1-13, wherein the cells comprise therapeutically useful cells for wound healing, preferably mesenchymal stem cells, adipose-derived stem cells, or bone-marrow derived stem cells.

15. The device according to any one of claims 1-14 for use in therapy.

16. The device according to any one of claims 1-14 for use in the treatment of wounds, preferably skin wounds or skin burns.

17. The device according to any one of claims 1-14 comprising adipose-derived stem cells or bone-marrow derived stem cells for use in preventing inflammation, immune rejection, or scar formation during recovery from dermal tissue damage.

18. A method of manufacturing a device according to any one of claims 1-14 comprising nanofibrillar polysaccharide arranged in a continuous arrangement, at least one side of the membrane comprising at least one patterned area comprising micro-scale recesses and/or protrusions, wherein the patterned area comprises a repeating pattern of units, wherein at least one dimension of a unit is from 1 μm to 500 μm along the plane of the membrane and wherein the method comprises the steps of

a. providing therapeutically useful cells;
b. absorbing a membrane comprising nanofibrillar polysaccharide, wherein the nanofibrillar polysaccharide is plant-derived nanofibrillar cellulose arranged in a continuous arrangement, at least one side of the membrane comprising at least one patterned area comprising micro-scale recesses and/or protrusions with an aqueous medium;
c. transferring the cells on the membrane; and
d. incubating the cells in conditions allowing attachment of the cells on the patterned area of the membrane and allowing maintenance or undifferentiated or differentiated growth of the cells.

19. The method according to claim 18, wherein the cells comprise autologous cells, allogeneic cells, stem cells, progenitor cells, precursor cells, connective tissue cells, epithelial cells, muscle cells, neuronal cells, endothelial cells, fibroblasts, keratinocytes, smooth muscle cells, stromal cells, mesenchymal cells, cord blood cells, embryonic stem cells, induced pluripotent cells, placental cells, bone marrow derived cells, immune system cells, hematopoietic cells, dendritic cells, hair follicle cells, chondrocytes, hybridoma cells, and combinations thereof.

**20.** The method according to any one of claims 18-20, wherein steps b and c are conducted simultaneously.

**Patentansprüche**

**1.** Vorrichtung umfassend:

eine Membran, die ein in kontinuierlicher Anordnung angeordnetes nanofibrilläres Polysaccharid umfasst, wobei mindestens eine Seite der Membran mindestens einen strukturierten Bereich umfasst, der mikroskalige Vertiefungen und/oder Überhöhungen aufweist, wobei das nanofibrilläre Polysaccharid pflanzenstämmige nanofibrilläre Cellulose umfasst und wobei der strukturierte Bereich ein sich wiederholendes Muster von Einheiten umfasst, wobei mindestens eine Abmessung einer Einheit 1 $\mu$m bis 500 $\mu$m entlang der Ebene der Membran beträgt, und
therapeutisch nützliche Zellen auf dem strukturierten Bereich der Membran.

**2.** Vorrichtung nach Anspruch 1, wobei die zahlenmittlere Dicke des strukturierten Bereichs 100 nm - 100 $\mu$m, bevorzugt 200 nm - 10 $\mu$m und höchstbevorzugt 1 - 10 $\mu$m beträgt.

**3.** Vorrichtung nach einem der Ansprüche 1 bis 2, wobei der strukturierte Bereich ein Wiederholungsmuster von Einheiten umfasst, die wechselseitig mit einer gemeinsamen Wand verbunden sind, die eine Breite von 10 nm - 10 $\mu$m, bevorzugt 100 nm - 1 $\mu$m, höchstbevorzugt 200 nm - 1 $\mu$m aufweist.

**4.** Vorrichtung nach einem der Ansprüche 1 bis 3, wobei die Membran eine Dicke von 1 - 300 $\mu$m, bevorzugt 10 - 100 $\mu$m, höchstbevorzugt 20 - 60 $\mu$m aufweist.

**5.** Vorrichtung nach einem der Ansprüche 1 bis 4, wobei die Membran 90 - 100 % Trockengewicht nanofibrilläres Polysaccharid, bevorzugt 95 - 100 % Trockengewicht nanofibrilläres Polysaccharid, bevorzugter 99 - 100 % Trockengewicht nanofibrilläres Polysaccharid umfasst.

**6.** Vorrichtung nach einem der Ansprüche 1 bis 5, wobei das nanofibrilläre Polysaccharid ein Derivat von pflanzenstämmiger Cellulose umfasst.

**7.** Vorrichtung nach einem der Ansprüche 1 bis 6, wobei das nanofibrilläre Polysaccharid mechanisch zerfasert wird.

**8.** Vorrichtung nach einem der Ansprüche 1 bis 7, wobei das nanofibrilläre Polysaccharid Polysaccharid-Nanofibrillen und/oder -Nanofibrillenbündel mit einem zahlenmittleren Durchmesser zwischen 1 und 500 nm, bevorzugt zwischen 2 und 200 nm, umfasst.

**9.** Vorrichtung nach einem der Ansprüche 1 bis 8, wobei die mikroskaligen Vertiefungen und/oder Überhöhungen Abmessungen aufweisen, die es den Zellen ermöglichen, die Vertiefungen der Membran aufzunehmen, und/oder den Zellen ermöglichen, sich an den Überhöhungen der Membran wesentlich anzuheften.

**10.** Vorrichtung nach einem der Ansprüche 1 bis 9, die ein im Inneren der Membran absorbiertes wässriges Medium umfasst, wobei das wässrige Medium umfasst: Wasser, steriles Wasser, gereinigtes Wasser, physiologische Kochsalzlösung, eine physiologische Pufferlösung, ein Kulturmedium, Ernährungsstoffe und/oder einen bioaktiven Stoff, oder Kombinationen derselben, wobei der bioaktive Stoff ausgewählt ist aus der Gruppe bestehend aus Eiweißen, Peptiden, Kohlenhydraten, Lipiden, Nukleinsäuren und Fragmenten derselben, antiviralen Verbindungen, entzündungshemmenden Verbindungen, antibiotischen Verbindungen wie antifungalen und antibakteriellen Verbindungen, zelldifferenzierenden Mitteln, Analgetika, Kontrastmitteln für die medizinischdiagnostische Bildgebung, Enzymen, Zytokinen, Narkosemitteln, Antihistaminen, auf das Immunsystem einwirkenden Mitteln, immunstimulierenden Mitteln, blutstillenden Mitteln, Hormonen, angiogenischen oder antiangiogenischen Mitteln, Neurotransmittern, therapeutischen Oligonukleotiden, viralen Partikeln, Vektoren, Wachstumsfaktoren, Retinoiden, Zelladhäsionsfaktoren, osteogenen Faktoren, Antikörpern, Antigenen, Peptiden, Zellen und deren Derivaten mit acellulärer Matrix.

**11.** Vorrichtung nach einem der Ansprüche 1 bis 10, wobei mindestens ein Teil der mindestens einen Seite der Membran mit einem Mittel zur Verstärkung der Zelladhäsion, das ausgewählt ist aus der Gruppe bestehend aus allen Arten von Extrazellularmatrix-Eiweißen wie Laminin, Fibronektin, Vitronektin, Kollagen Typ I, Kollagen Typ II und Kollagen Typ IV und/oder Kombinationen oder Fraktionen derselben oder komplexen Gemischen, beschichtet oder chemisch verbunden ist.

**12.** Vorrichtung nach einem der Ansprüche 1 bis 11, darüber hinaus umfassend: eine oder mehrere Schichten, die sich über mindestens einen Teil der biomedizinischen Vorrichtung, wie über den zentralen oder peripheren Bereich der Vorrichtung, erstrecken, wobei die eine oder mehreren Schichten ausgewählt sind aus einer Stützschicht, einer Trägerschicht, einer feuchtigkeitsbindenden Schicht, einer feuchtigkeitsabsorbierenden Schicht, einer Feuchtigkeitssperrschicht, einer Gassperrschicht, einer geruchsabsorbierenden Schicht, einer arzneimittelhaltigen Schicht, einer adhäsiven Schicht und/oder einer mucoadhäsiven Schicht.

**13.** Vorrichtung nach einem der Ansprüche 1 bis 12, wobei die Zellen umfassen: autologe Zellen, allogene Zellen, Stammzellen, Progenitorzellen, Vorläuferzellen, Bindegewebszellen, Epithelzellen, Muskelzellen, neuronale Zellen, Endothelzellen, Fibroblasten, Keratinozyten, glatte Muskelzellen, Stromazellen, Mesenchymzellen, Nabelschnurblutzellen, embryonale Stammzellen, induzierte pluripotente Zellen, Plazentazellen, knochenmarkstämmige Zellen, Immunsystemzellen, hämatopoetische Zellen, dendritische Zellen, Haarfollikelzellen, Chondrozyten, Hybridomzellen und/oder Kombinationen derselben.

**14.** Vorrichtung nach einem der Ansprüche 1 bis 13, wobei die Zellen therapeutisch nützliche Zellen für die Wundheilung, bevorzugt mesenchymale Stammzellen, fettstämmige Stammzellen oder knochenmarkstämmige Stammzellen, umfassen.

**15.** Vorrichtung nach einem der Ansprüche 1 bis 14 zur Verwendung in der Therapie.

**16.** Vorrichtung nach einem der Ansprüche 1 bis 14 zur Verwendung bei der Behandlung von Wunden, bevorzugt Hautwunden oder Hautverbrennungen.

**17.** Vorrichtung nach einem der Ansprüche 1 bis 14, umfassend: fettstämmige Stammzellen oder knochenmarkstämmige Stammzellen zur Verwendung bei der Verhinderung von Entzündung, Immunabstoßung oder Narbenbildung während der Genesung von Hautgewebeschädigungen.

**18.** Verfahren zur Herstellung einer Vorrichtung nach einem der Ansprüche 1 bis 14, die ein in kontinuierlicher Anordnung angeordnetes nanofibrilläres Polysaccharid umfasst, wobei mindestens eine Seite der Membran mindestens einen strukturierten Bereich umfasst, der mikroskalige Vertiefungen und/oder Überhöhungen aufweist, wobei der strukturierte Bereich ein sich wiederholendes Muster von Einheiten umfasst, wobei mindestens eine Abmessung einer Einheit 1 $\mu$m bis 500 $\mu$m entlang der Ebene der Membran beträgt und wobei das Verfahren die Schritte umfasst:

a. Bereitstellen von therapeutischen nützlichen Zellen;
b. Absorbieren eines wässrigen Mediums in einer Membran, die nanofibrilläres Polysaccharid umfasst, wobei es sich beim nanofibrillären Polysaccharid um pflanzenstämmige nanofibrilläre Cellulose handelt, die in einer kontinuierlichen Anordnung angeordnet ist, wobei mindestens eine Seite der Membran mindestens einen strukturierten Bereich umfasst, der mikroskalige Vertiefungen und/oder Überhöhungen aufweist,
c. Übertragen der Zellen auf die Membran; und
d. Inkubieren der Zellen unter Bedingungen, die ein Anheften der Zellen am strukturierten Bereich der Membran ermöglichen und ein Aufrechterhalten oder undifferenziertes oder differenziertes Wachstum der Zellen ermöglichen.

**19.** Verfahren nach Anspruch 18, wobei die Zellen umfassen: autologe Zellen, allogene Zellen, Stammzellen, Progenitorzellen, Vorläuferzellen, Bindegewebszellen, Epithelzellen, Muskelzellen, neuronale Zellen, Endothelzellen, Fibroblasten, Keratinozyten, glatte Muskelzellen, Stromazellen, Mesenchymzellen, Nabelschnurblutzellen, embryonale Stammzellen, induzierte pluripotente Zellen, Plazentazellen, knochenmarkstämmige Zellen, Immunsystemzellen, hämatopoetische Zellen, dendritische Zellen, Haarfollikelzellen, Chondrozyten, Hybridomzellen und/oder Kombinationen derselben.

**20.** Verfahren nach einem der Ansprüche 18 bis 20, wobei die Schritte b und c gleichzeitig ausgeführt werden.

**Revendications**

**1.** Dispositif comprenant

une membrane comprenant un polysaccharide nanofibrillaire disposé dans un agencement continu, au moins un côté de ladite membrane comprenant au moins une zone modelée comprenant des évidements et/ou protubérances à l'échelle micrométrique, ledit polysaccharide nanofibrillaire comprenant une cellulose nanofibrillaire dérivée de plantes et ladite zone modelée comprenant un motif répété d'unités, au moins une dimension d'une unité étant de 1 μm à 500 μm le long du plan de la membrane, et
des cellules thérapeutiquement utiles sur la zone modelée de la membrane.

2. Dispositif selon la revendication 1, dans lequel l'épaisseur moyenne en nombre de la zone modelée est de 100 nm - 100 μm, préférablement de 200 nm - 10 μm et le plus préférablement de 1 - 10 μm.

3. Dispositif selon l'une des revendications 1 à 2, dans lequel la zone modelée comprend un motif répété d'unités reliées entre elles par une paroi commune ayant une largeur de 10 nm - 10 μm, préférablement de 100 nm - 1 μm, le plus préférablement de 200 nm - 1 μm.

4. Dispositif selon l'une des revendications 1 à 3, dans lequel la membrane a une épaisseur de 1 - 300 μm, préférablement de 10 - 100 μm, le plus préférablement de 20 - 60 μm.

5. Dispositif selon l'une des revendications 1 à 4, dans lequel la membrane comprend 90 - 100 % en poids sec de polysaccharide nanofibrillaire, préférablement 95 - 100 % en poids sec de polysaccharide nanofibrillaire, plus préférablement 99 - 100 % en poids sec de polysaccharide nanofibrillaire.

6. Dispositif selon l'une des revendications 1 à 5, dans lequel le polysaccharide nanofibrillaire comprend un dérivé d'une cellulose dérivée de plantes.

7. Dispositif selon l'une des revendications 1 à 6, dans lequel le polysaccharide nanofibrillaire est désintégré mécaniquement.

8. Dispositif selon l'une des revendications 1 à 7, dans lequel le polysaccharide nanofibrillaire comprend des nanofibrilles et/ou faisceaux de nanofibrilles de polysaccharide ayant un diamètre moyen en nombre d'entre 1 et 500 nm, préférablement d'entre 2 et 200 nm.

9. Dispositif selon l'une des revendications 1 à 8, dans lequel les évidements et/ou protubérances à l'échelle micrométrique présentent des dimensions permettant aux cellules de loger les évidements de la membrane et/ou permettant aux cellules de s'attacher essentiellement aux protubérances de la membrane.

10. Dispositif selon l'une des revendications 1 à 9, comprenant un médium aqueux absorbé à l'intérieur de la membrane, ledit médium aqueux comprenant de l'eau, de l'eau stérile, de l'eau purifiée, une solution saline physiologique, un tampon physiologique, un milieu de culture, des agents nutritionnels et/ou un agent bioactif, ou des combinaisons de ceux-ci, ledit agent bioactif étant choisi dans le groupe constitué par les protéines, les peptides, les glucides, les lipides, les acides nucléiques et leurs fragments, les composés antiviraux, les composés anti-inflammatoires, les composés antibiotiques tels que les composés antifongiques et antibactériens, les agents de différenciation cellulaire, les analgésiques, les agents de contraste pour l'imagerie diagnostique médicale, les enzymes, les cytokines, les anesthésiques, les antihistamines, les agents qui agissent sur le système immunitaire, les agents immunostimulateurs, les agents hémostatiques, les hormones, les agents angiogéniques ou anti-angiogéniques, les neurotransmetteurs, les oligonucléotides thérapeutiques, les particules virales, les vecteurs, les facteurs de croissance, les rétinoïdes, les facteurs d'adhésion cellulaire, les facteurs ostéogéniques, les anticorps, les antigènes, les peptides, les cellules et leurs dérivés comprenant une matrice acellulaire.

11. Dispositif selon l'une des revendications 1 à 10, dans lequel au moins une partie dudit au moins un côté de la membrane est revêtue d'un, ou chimiquement liée à un, agent pour améliorer l'adhésion cellulaire choisi dans le groupe constitué par toutes sortes de protéines de matrice extracellulaire telles que la laminine, la fibronectine, la vitronectine, le collagène de type I, le collagène de type II et le collagène de type IV et/ou leurs combinaisons ou fractions ou mélanges complexes.

12. Dispositif selon l'une des revendications 1 à 11, comprenant en plus une ou plusieurs couches s'étendant sur au moins une partie du dispositif biomédical, telle que la partie centrale ou périphérique du dispositif, lesdites une ou plusieurs couches étant choisies parmi une couche de support, une couche d'appui, une couche de rétention d'humidité, une couche absorbant l'humidité, une couche barrière contre l'humidité, une couche barrière contre les gaz, une couche absorbant les odeurs, une couche contenant un médicament, une couche adhésive et/ou une couche mucoadhésive.

13. Dispositif selon l'une des revendications 1 à 12, dans lequel les cellules comprennent les cellules autologues, les cellules allogéniques, les cellules souches, les cellules progénitrices, les cellules précurseurs, les cellules de tissu conjonctif, les cellules épithéliales, les cellules musculaires, les cellules neuronales, les cellules endothéliales, les fibroblastes, les kératinocytes, les cellules musculaires lisses, les cellules stromales, les cellules mésenchymateuses, les cellules de sang de cordon, les cellules souches embryonnaires, les cellules pluripotentes induites, les cellules placentaires, les cellules dérivées de moelle osseuse, les cellules de système immunitaire, les cellules hématopoïétiques, les cellules dendritiques, les cellules des follicules pileux, les chondrocytes, les cellules d'hybridomes et/ou des combinaisons de celles-ci.

14. Dispositif selon l'une des revendications 1 à 13, dans lequel les cellules comprennent des cellules thérapeutiquement utiles pour la guérison des plaies, préférablement des cellules souches mésenchymateuses, des cellules souches adipocytaires ou des cellules souches dérivées de moelle osseuse.

15. Dispositif selon l'une des revendications 1 à 14 pour l'utilisation en thérapeutique.

16. Dispositif selon l'une des revendications 1 à 14 pour l'utilisation dans le traitement de plaies, préférablement de plaies de la peau ou brûlures de la peau.

17. Dispositif selon l'une des revendications 1 à 14 comprenant des cellules souches adipocytaires ou des cellules souches dérivées de moelle osseuse pour l'utilisation dans la prévention d'inflammations, du rejet immunitaire ou de la formation de cicatrices lors du rétablissement après une lésion du tissu cutané.

18. Procédé de fabrication d'un dispositif selon l'une des revendications 1 à 14 comprenant un polysaccharide nanofibrillaire disposé dans un agencement continu, au moins un côté de ladite membrane comprenant au moins une zone modelée comprenant des évidements et/ou protubérances à l'échelle micrométrique, ladite zone modelée comprenant un motif d'unités répétées, au moins une dimension d'une unité étant de 1 $\mu$m à 500 $\mu$m le long du plan de la membrane et ledit procédé comprenant les étapes consistant à

   a. fournir des cellules thérapeutiquement utiles ;
   b. faire absorber un médium aqueux par une membrane comprenant un polysaccharide nanofibrillaire, ledit polysaccharide nanofibrillaire étant une cellulose nanofibrillaire dérivée de plantes disposée dans un agencement continu, au moins un côté de ladite membrane comprenant au moins une zone modelée comprenant des évidements et/ou protubérances à l'échelle micrométrique,
   c. transférer les cellules sur la membrane ; et
   d. incuber les cellules dans des conditions permettant l'attachement des cellules sur la zone modelée de la membrane et permettant le maintien ou la croissance indifférenciée ou différenciée des cellules.

19. Procédé selon la revendication 18, dans lequel les cellules comprennent les cellules autologues, les cellules allogéniques, les cellules souches, les cellules progénitrices, les cellules précurseurs, les cellules de tissu conjonctif, les cellules épithéliales, les cellules musculaires, les cellules neuronales, les cellules endothéliales, les fibroblastes, les kératinocytes, les cellules musculaires lisses, les cellules stromales, les cellules mésenchymateuses, les cellules de sang de cordon, les cellules souches embryonnaires, les cellules pluripotentes induites, les cellules placentaires, les cellules dérivées de moelle osseuse, les cellules de système immunitaire, les cellules hématopoïétiques, les cellules dendritiques, les cellules des follicules pileux, les chondrocytes, les cellules d'hybridomes et/ou des combinaisons de celles-ci.

20. Procédé selon l'une des revendications 18 à 20, dans lequel les étapes b et c sont réalisées simultanément.

FIG. 1

FIG. 2

FIG. 3

FIG. 4

A

Elec_Mat0158        2013.06.11   10:46      D1.9   x100      1 mm

B

Elec_Mat0159        2013.06.11   10:47      D1.9   x400      200 um

C

Elec_Mat0160        2013.06.11   10:48      D1.9   x1.2k      50 um

D

Elec_Mat0330        2013.06.19   09:58      D2.1   x400      200 um

FIG. 5

A

Elec_Mat0152    2013.06.11  10:20    D1.9  x100    1 mm

B

Elec_Mat0153    2013.06.11  10:21    D1.9  x400    200 um

C

Elec_Mat0155    2013.06.11  10:25    D1.9  x1.2k    50 um

D

Elec_Mat0333    2013.06.19  10:11    D2.0  x400    200 um

FIG. 6

FIG. 7

A

B

C

D

FIG. 8

FIG. 9

FIG. 10

NON-TREATED      LAMININ      CELLSTART

FIG. 11

FIG. 12

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0103750 A
- WO 2013171373 A
- WO 2012056109 A
- EP 06742940 A

**Non-patent literature cited in the description**

- **RODRIGUEZ K. et al.** Electrospun nanofibrous cellulose scaffolds with controlled microarchitecture. *Carbohydrate Polymers,* 2013
- A guide to biological skin substitutes. *British Journal of Plastic Surgery,* 2002, vol. 55, 185-193
- **ESCOBEDO-LUCEA et al.** A Xenogeneic-Free Protocol for Isolation and Expansion of Human Adipose Stem Cells for Clinical Uses. *Plos One,* 09 July 2013
- **ESCOBEDO-LUCEA et al.** *Plos One,* 2013